# EUROPEAN PATENT SPECIFICATION

(11) **EP 2 152 153 B1**
(45) Date of publication and mention of the grant of the patent: **02.09.2015**
(21) Application number: 08738295.8
(22) Date of filing: 01.05.2008
(51) Int. Cl.: A61B 5/00, A61B 8/00, A61B 5/01, A61B 5/05, A61B 5/053, A61B 5/055, A61B 5/06

(54) **CLEAN MARGIN ASSESSMENT TOOL**
BEWERTUNGSWERKZEUG FÜR SAUBERE RÄNDER
INSTRUMENT D'ÉVALUATION D'UN BORD DE TISSU SAIN

(30) Priority: 01.05.2007 US 743028; 07.05.2007 US 745334
(43) Date of publication of application: 17.02.2010
(73) Proprietor: Dune Medical Devices Ltd., 38900 Caesarea (IL)
(72) Inventor: HASHIMSHONY, Dan, 37808 Givat Ada (IL); COHEN, Gil, 93706 Jerusalem (IL); GELTNER, Iddo, 46588 Herzlia (IL)
(74) Representative: Becker Kurig Straus
(86) International application number: PCT/IL2008/000594
(87) International publication number: WO 2008/132750

(56) References cited:
- WO-A-2006/072947
- WO-A-2007/015255
- AU-B2- 688 352
- US-A1- 2005 021 019
- US-A1- 2006 253 107

## Description

### FIELD AND BACKGROUND OF THE INVENTION

The present invention relates generally to a medical tool and method for use in removal of abnormal tissues (e.g. tumors) from a subject's body.

When dealing with removal of an abnormal tissue portion, a layer of healthy tissue must enclose the abnormal (cancerous) tissue portion, to ensure that all the malignancy has been removed. This layer is often referred to as a "clean margin". Although generally dependent on the size and shape of the tumor that is being removed, a desired depth of the clean margin may range from 1 cell layer, or about 40 microns, to 10 mm.

Typically the surgeon uses a scalpel and (or) an electrosurgical cutting device to remove a tissue portion enclosing the tumor, in one piece, and manage bleeding. The removed portion is transported to the pathologist, who samples the margins, histologically, at specific and suspicious points, for example, at one or a few representative points on each face of the portion, to assess whether the cancer has been completely removed from the body. If the pathologists deems that cancer cells are too close to the edge of the portion, i.e., if he deems the margin infected, a re-excision is recommended, and the patient must undergo a second surgical procedure to remove more of the tissue.

Various techniques have been developed for characterizing and removing tissue from a subject's body. Such techniques are disclosed, for example, in US 6,813,515; US 7,184,824; US 7,082,325, all assigned to the assignee of the present application.

### GENERAL DESCRIPTION

The present invention provides a novel medical device and method for examining a tissue portion, e.g. to manage a tissue removal process. The tissue portion under examination may be that removed from a subject's body or tissue inside the subject's body. The technique of the present invention is aimed at determining whether the removed tissue portion has clean margin (healthy tissue), or whether the tissue portion that is to be removed as being abnormal has clean margin and the surrounding of said tissue portion has no abnormal tissues. The device of the present invention includes one or more tissue-type sensors (sensors adapted for characterizing the tissue) for identifying clean margin within a periphery region of a tissue portion under examination. This enables removal of said tissue portion and/or removal of tissues adjacent to (surrounding) said tissue portion. A standard device for use in monitoring a tissue is described in US 2006/0253107 disclosing the preamble of claim 1.

According to a broad aspect of the invention, there is provided a device for use in monitoring a tissue, the device being configured for holding and characterizing a tissue portion during the monitoring, said device comprising: a housing configured for receiving and holding the tissue portion by an inner side or an outer side of the housing; a tissue-type sensor unit mounted on either one or both of the inner and outer sides of the housing and configured and operable for characterizing the tissue portion being held by the housing.

Preferably, the sensor unit includes an array of sensing elements. The sensor(s) may be attached to either the inner or the outer side of the housing. The sensor elements may be distributed within the surface of the housing.

The device may comprise a robotic arm for rotating the sensor unit with respect to the housing, and/or for rotating the housing with respect to the sensor unit.

The housing has a body having one of the following configurations: a rigid body, a flexible body, a stretchable body, and an expansible body. The body of the housing may have a shape of an anatomical feature.

The device may comprise at least one signal communication bundle for transmitting and receiving signals from and to the sensor unit.

In some embodiments, the housing has a portion thereof configured such that, when brought in contact with the tissue, to be enclosed by a portion of said tissue, e.g. by a continuous surface of the tissue portion, e.g. in the form of a cavity or a closed lumen. The device may include an attachment mechanism configured to attach the tissue portion to the housing, for example by causing the tissue to wrap the surface of the portion of the housing. The attachment mechanism may comprise a vacuum system (source). Accordingly, in some embodiments, the device comprises connector to a vacuum source. The housing may comprise one or more openings to enable vacuum communication between the tissue and the vacuum source.

The device of the present invention may be configured for monitoring the tissue while in a subject's body (e.g. before or during the removal of the tissue portion from the body), or for monitoring the removed tissue outside the subject's body.

According to yet another broad aspect of the invention, there is provided a system for use in monitoring a tissue. The system comprises the above described device; and a computerized system. The latter is connectable to the device, and comprises: an analyzer utility configured and operable for analyzing data generated by the tissue-type sensor unit, determining whether a clean margin of healthy tissue exists in a periphery region of the tissue portion, and generating data indicative of the analysis results; and an output device, which provides output data corresponding to said data generated by the analyzer utility.

### BRIEF DESCRIPTION OF THE DRAWINGS

The invention is herein described, by way of example only, with reference to the accompanying drawings. With specific reference now to the drawings in detail, it is stressed that the particulars shown are by way of example and for purposes of illustrative discussion of the preferred embodiments of the present invention only, and are presented in the cause of providing what is believed to be the most useful and readily understood description of the principles and conceptual aspects of the invention. In this regard, no attempt is made to show structural details of the invention in more detail than is necessary for a fundamental understanding of the invention, the description taken with the drawings making apparent to those skilled in the art how the several forms of the invention may be embodied in practice.

In the drawings:
**FIGs. 1a** - **1f** schematically illustrate the application of an integrated tool for clean-margin assessment to a soft tissue that contains a cancerous tissue within and the principles of clean-margin assessment, in accordance with the present invention;
**FIGs. 2a - 2c** schematically illustrate an isometric view, a frontal view, and a cross-sectional view of the integrated tool for clean-margin assessment, in accordance with the present invention;
**FIG. 3** schematically illustrates an ultrasound distance-measuring sensor of the integrated tool for clean-margin assessment, in accordance with the present invention;
**FIGs. 4a - 4d** further illustrate the operational manner of the integrated tool for clean-margin assessment, in accordance with the present invention;
**FIGs. 5a - 5c** further illustrate the operational manner of the integrated tool for clean-margin assessment, in accordance with the present invention;
**FIG. 6** schematically illustrates an overall system for clean-margin assessment, in accordance with the present invention;
**FIGs. 7a - 7d** schematically illustrate the integrated tool for clean-margin assessment, which further includes a retractable knife, in accordance with a preferred embodiment of the present invention;
**FIGs. 8a - 8b** schematically illustrate the integrated tool for clean-margin assessment, operative with a frame for fixing a soft tissue, in accordance with a preferred embodiment of the present invention;
**FIGs. 9a and 9b** schematically illustrate the integrated tool for clean-margin assessment, wherein the tissue-type sensor is formed as a horn antenna, for RF or MW, in accordance with still another embodiment of the present invention;
**FIGs. 10a and 10b** schematically illustrate the integrated tool for clean-margin assessment, wherein the tissue-type sensor is formed as an optical sensor, in accordance with yet another embodiment of the present invention;
**FIGs. 11a and 11b** schematically illustrate the integrated tool for clean-margin assessment, wherein the tissue-type sensor is formed as an MRI sensor, in accordance with yet another embodiment of the present invention;
**FIGs. 12a and 12b** schematically illustrate the integrated tool for clean-margin assessment, wherein the distance-measuring sensor is formed as a strain gauge, in accordance with still another embodiment of the present invention;
**FIGs. 13a and 13b** schematically illustrate the integrated tool for clean-margin assessment, wherein the distance-measuring sensor is formed as a pressure sensor, in accordance with still another embodiment of the present invention;
**FIGs. 14a** **14b** illustrate, in flowchart forms, surgical methods of tumor removal, using the integrated tool for clean-margin assessment, in accordance with embodiments of the present invention;
**FIGs. 15a to 15c** illustrate the principles of a clean margin technique in accordance with the present invention, where FIGs. 15a and 15b show the top and cross sectional views, respectively, of a tissue part including a tissue portion under characterization or from which a tissue portion have been removed; and FIG. 15c shows more specifically a peripheral region or surrounding region of said tissue portion;
**FIGs. 16a** **and** **16b** illustrate flowcharts of a method of the present invention for providing a clean margin of healthy tissue around a malignant tumor or abnormal tissue;
**FIG. 17** exemplifies a device of the present invention for holding and characterizing a tissue or an anatomical feature during a clean-margin assessment process; and
**FIGs. 18a and 18b** illustrate a side view and a three dimension view, respectively, of a sensor array frame structure.

### DESCRIPTION OF EMBODIMENTS

The device of the present invention is a probe having a tissue-type sensor, for determining the tissue type at a near zone volume of a tissue surface. The device may also include a distance-measuring sensor, for determining the distance to an interface with another tissue type. This may for example be an integrated tool including the probe and the distance-measuring sensor. The device is operable for (i) confirming an existence of a clean margin of healthy tissue around a malignant tumor, which is being removed, and (ii) determining the width of the clean margin, wherein both are performed in real time, while the malignant tumor is being removed.

The tissue-type sensor may be selected from the following: a sensor for tissue electromagnetic properties, a dielectric sensor, an impedance sensor, a sensor for optical fluorescence spectroscopy, a sensor for optical reflectance spectroscopy, an MRI sensor, an RF sensor, an MW sensor, a temperature sensor, and infrared thermography sensor, or another tissue-characterization sensor, as known. The distance-measuring sensor may include at least one of the following: an ultrasound transducer, an MRI probe, an invasive needle with a strain or pressure gauge, or another tissue distance measuring sensor, as known. The integrated tool may further include a position tracking device and an incision instrument. The soft tissue may be held within a fixed frame, while the tumor is being removed. Additionally a method for malignant tumor removal is provided, comprising, fixing the soft tissue within a frame, performing imaging with the hand-held, integrated tool, from a plurality of locations and orientations around the soft tissue, reconstructing a three-dimensional image of the soft tissue and the tumor within, defining a desired clean margin on the reconstructed image, calculating a recommended incision path, displaying the recommended path on the reconstructed image, and cutting the tissue while determining its type, at the near zone volume of the incision surface, by the hand-held integrated tool. The method may further include continuously imaging with the cutting, continuously correcting the reconstructed image and the recommended incision path, and continuously determining the tissue type, at the near zone volume of the incision surface.

Before explaining at least one embodiment of the invention in detail, it should be understood that the invention is not limited in its application to the details of construction and the arrangement of the components set forth in the following description or illustrated in the drawings. The invention is capable of other embodiments or of being practiced or carried out in various ways. Also, it is to be understood that the phraseology and terminology employed herein is for the purpose of description and should not be regarded as limiting.

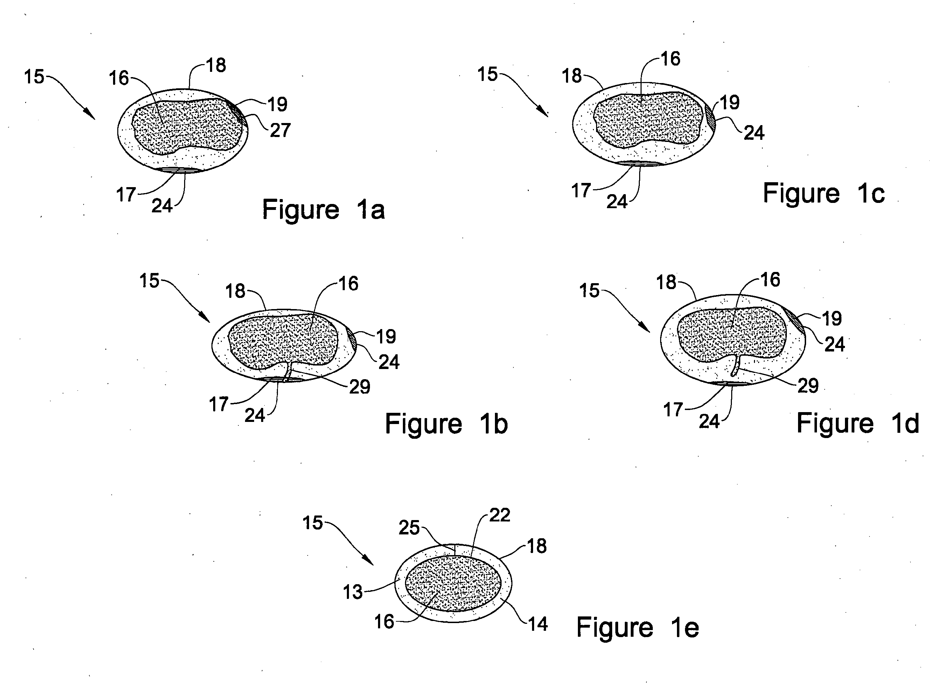

Referring now to the drawings, **Figures 1a - 1f** schematically illustrate the principles of clean margin assessment and the application of a hand-held, integrated tool 10 for clean-margin assessment, in accordance with some embodiments of the present invention.

The principles of clean margin assessment may be understood using the examples of **Figures 1a - 1d****.** These illustrate tissue portions **15** which have been removed from the body. These portions include a first tissue type of healthy tissue **14,** enclosing or partly enclosing a second tissue type of cancerous or otherwise abnormal tissue **16.** A tissue surface **18,** which is generally the incision surface, bounds each of the tissue portions **15.**

However, it will be appreciated that the tissue surface **18** may be a skin, a body lumen, or an incision surface; either an arbitrary incision surface, or an incision surface contouring an organ and an anatomical feature.

As seen in **Figure 1a**, the incision surface **18** has a positive margin **27** at a location **19.** This means that cancerous or otherwise abnormal cells have reached the surface **18** or the near zone volume of the surface **18,** at the location **19.** This may happen when the incision was performed right through the cancerous or abnormal second tissue type **16.** Alternatively, this may happen when the incision is performed at the interface between the first and second tissue types, **14** and **16.**

The near zone at the tissue surface **18** is at least one cell layer in thickness, and preferably several cell layers in thickness. In practice, it may range from about 100 microns to about 500 microns.

Thus, the positive margin **27** may be defined as a situation where the tissue surface **18,** or the near zone at the tissue surface **18,** contains at least one cancerous cell.

**Figure 1a** further illustrates a clean margin at a location **17,** where the tissue surface **18,** or the near zone at the tissue surface **18,** contains no cancerous cells, and thus has a clean margin **24.**

**Figure 1b** illustrates another example of the positive margin **27,** this time at the location **17.** The positive margin of Figure 1b, however, is due to a shoot **29,** which stems from the second tissue type **16** and which reaches to the surface **18.**

By contrast, **Figures 1c and 1d** illustrate examples of tissue portions **15** that have been excised with clean margins **24,** at all locations.

**Figure 1e** illustrates a model for clean margin assessment, showing the second, cancerous tissue type **16** and a layer of a tissue **13,** surrounding it. The tissue **13** may be a healthy tissue, but may be partly cancerous or otherwise abnormal. The aim in characterizing the tissue surface **18** is to determine the type of the tissue **13** at various locations along the surface **18.** Additionally, when the tissue surface **18** is characterized as the clean margin **24,** a depth **25** to an interface **22** with the second tissue type **16,** may be defined. While a sufficient depth may be realized when the depth **25** is only 1 cell layer in thickness, or about 40 microns, it is generally desired that the depth **25** be between about 0.1 and 10 mm.

It will be appreciated that other dimensions for the depth 25 of the clean margin may be desired and may depend on the size and type of the cancerous tumor, forming the second tissue type **16.**

During a surgical operation, for the removal of a cancerous tumor, in a breast for example, it is important to ensure that the incision is made through a healthy tissue, so that all the cancerous tissue is completely contained within the healthy tissue that is being removed. Thus, the indicated need is to remove a tissue portion **15** as shown in Fig. 1e, such that:
i. the cut is made through the first tissue type **14** of healthy tissue, so as to completely contain the second tissue type **16** within;
ii. the depth **25** of the clean margin **24** of the first tissue type **14** is sufficient.

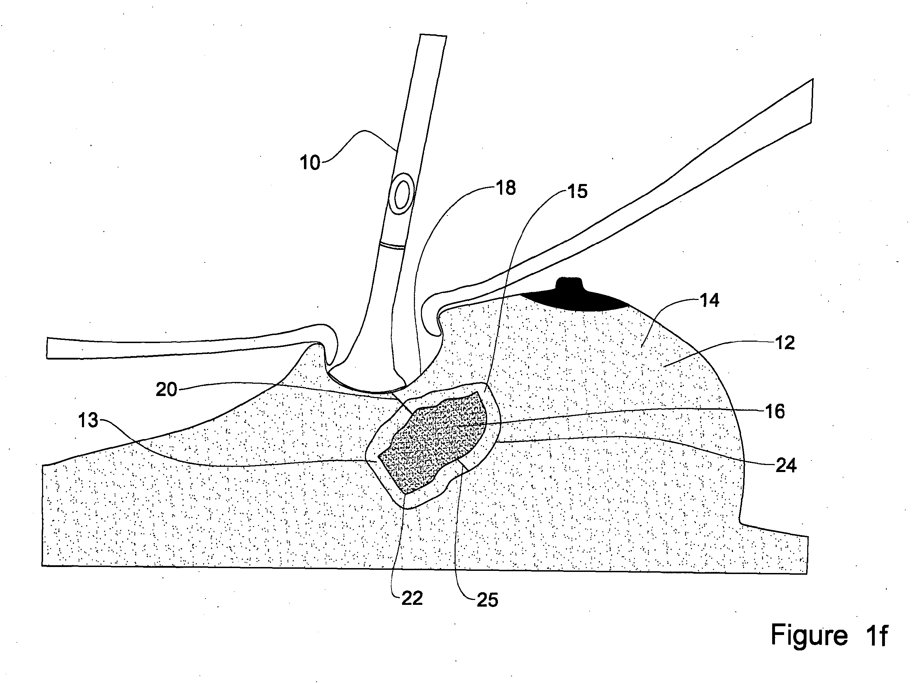

In accordance with the present invention, as illustrated by **Figure 1f****,** this indicated need is fulfilled by the hand-held, integrated tool **10** for clean-margin assessment, by:
i. a first sensor for characterizing the near zone volume of the tissue surface 18, to ensure that it is of the first tissue type **14** of healthy tissue; and
ii. a second sensor for measuring the depth **25** of the clean margin **24,** to verify that there is sufficient depth between the tissue surface **18** and the interface **22,** which bounds the second tissue type **16.**

It is important to note that either sensor alone would be insufficient for the task, since it would not give sufficient information about both the character of the near zone volume of the tissue surface and the depth of the clean margin. The prior art for example, includes methods for determining the depth of the margin but lacks the ability to characterize the tissue of which the margin is formed, so as to ensure that the margin which is measured is *clean.* It is by this aspect, of both characterizing the tissue of the margin and measuring its depth, that the present invention overcomes the shortcomings of prior art configurations.

**Figure 1f** further illustrates the application of the hand-held, integrated tool 10 for clean-margin assessment, to a tissue **12.** The tissue **12** includes the healthy tissue, which forms the first tissue type **14.** Additionally, the tissue **12** includes the cancerous or otherwise abnormal tissue, which forms the second tissue type **16,** enclosed within the first tissue type **14.**

In the example of **Figure 1f**, the integrated tool **10** determines that a distance **20** between the tissue surface **18** and the interface **22,** which bounds the second tissue type **16,** is about twice as much as the desired depth **25** of the clean margin **24.** In that case, a surgeon may decide to approach the second tissue type 16 further, in order to keep the size of the portion for removal minimal.

It will be appreciated that the integrated tool **10** may be further used to characterize additional tissue types and determine the distances between their interfaces. The various tissue types may include bone tissue, fat tissue, muscle tissue, cancerous tissue, or blood clot tissue.

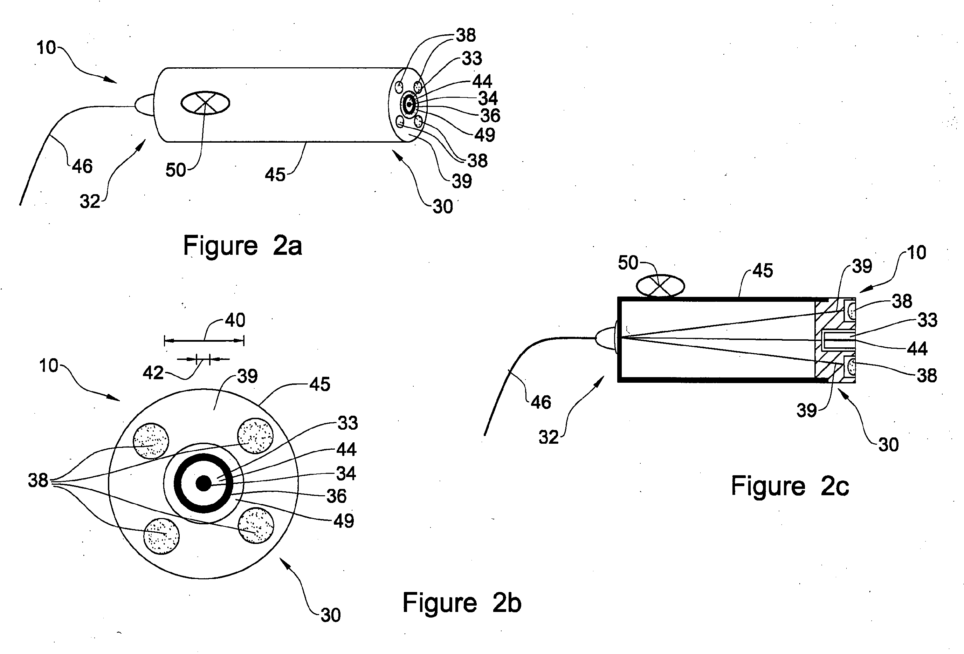

Referring further to the drawings, **Figures 2a - 2c** schematically illustrate an isometric view, a proximal view, with respect to the tissue **12,** and a cross-sectional view of the integrated tool **10** for clean-margin assessment, in accordance with a first embodiment of the present invention.

The integrated tool **10** has a proximal end **30** and a distal end **32,** with respect to the surface **18** (Figure 1). In accordance with the preferred embodiment of the present invention, a tissue-type sensor **33** determines the characteristics of the tissue in the near zone volume of the surface **18,** for example, whether fat, muscle, bone, healthy, cancerous, or otherwise abnormal. Additionally, a distance-measuring sensor **38** measures the distance **20** from the surface **18** to the interface **22** with the second tissue type **16.**

In accordance with the first embodiment of the present invention, the tissue-type sensor **33** measures the electrical properties of the tissue type **13.** By comparing the results with known tissue properties, the characteristic of the tissue type **13** is determined.

For example, the tissue-type sensor **33** may be constructed as a coaxial cable **44,** having an inner electrode **34** and an outer electrode **36,** which together form the sensor **33.** The outer electrode **36** may be grounded.

Further in accordance with the first embodiment of the present invention, the distance-measuring sensor **38** is at least one ultrasound transducer **38.**

Preferably, the coaxial cable **44** is located within an overall structure **45.** The distance-measuring sensor **38,** such as the at least one ultrasound transducer **38** is also mounted on the structure **45,** for example, along side the tissue-type sensor **33.**

Additionally, the distance-measuring sensor **38** may be formed of at least two ultrasound transducers **38,** one operating as a transmitter and the other as a receiver. The advantage there is that the instrumentation dead time is shorter.

Furthermore, the distance-measuring sensor **38** may be formed as an array of ultrasound transducers **38,** for providing steering and focusing capabilities, as known.

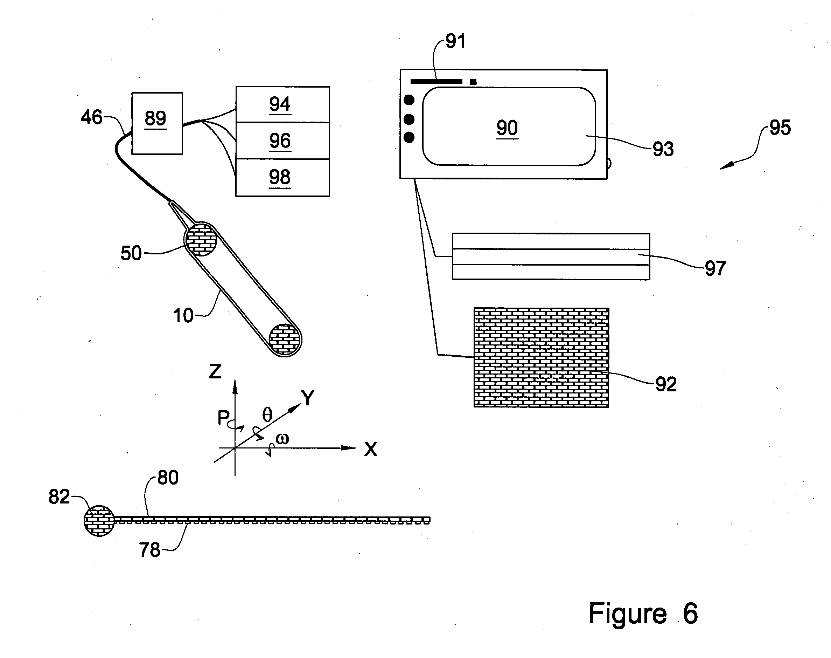

Signals from the tissue-type sensor **33** and the distance-measuring sensor **38** are transferred for analysis through a cable **46** to a computerized system **95,** described hereinbelow in conjunction with Figure 6.

Preferably, the inner electrode **34** has a diameter **40** of between about 0.2 and 1.5 mm, and the outer electrode **36** has an inner diameter **42** of between about 3.0 and 10.0 mm, and is about 0.5 mm thick. Additionally, the outer electrode **36** is covered with an insulating sheath **49** made of an insulating material, for example, Teflon. It will be appreciated that other dimensions, which may be larger or smaller, may similarly be used. The sensors **38** and **33** may be encased in a filler material **39,** for example epoxy, which may be formed as a plug that fits into the structure **45,** for example, as shown in **Figure 2c****.**

Preferably, the ultrasound transducer **38** operates at a frequency range of between about 0.5 MHz and about 40 MHz. It has an accuracy of about 3mm, when operating at the lower range of 0.5 MHz, and an accuracy of about 40 micron, when operating at the higher range of 40 MHz.

The integrated tool **10** may further include a position-tracking device 50, for example, the miniBIRD^{®} 500 or the miniBIRD^{®} 800, which are miniaturized magnetic tracking systems having six degrees of freedom and using sensors, which are merely 5 mm wide, produced by Ascension Technology Corporation, P.O. Box 527 Burlington, VT 05402, USA. They are described in http://www.ascension-tech.com/products/minibird.php, downloaded on March 15, 2005. The position-tracking device 50 may provide the coordinates of the ultrasound measurements, thus enabling a three-dimensional image reconstruction of the ultrasound.

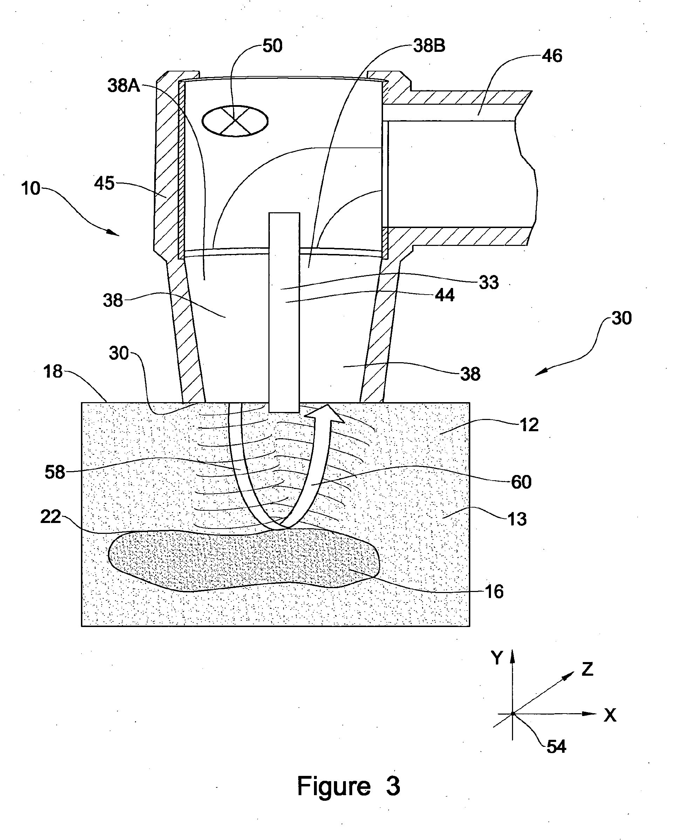

Referring further to the drawings, **Figure 3** schematically illustrates the ultrasound distance-measuring sensor **38** of the integrated tool **10,** in operation, in accordance with the present invention.

For operation, the proximal end **30** of the integrated tool **10** is brought proximally to the tissue surface **18,** of the tissue **12,** so as to make contact or near contact with it. The tissue **12** includes the first tissue type **14** of healthy tissue, preferably at the outer portion thereof, and the second tissue type **16** of abnormal tissue, enclosed by the first tissue type **14** of healthy tissue, with tissue **13,** which is suspicious as possibly containing cancerous or otherwise abnormal tissue, surrounding the second tissue type **16.** Preferably, tissue **16** is bounded by the interface **22.**

Preferably, at least two ultrasound transducers **38** are used, **38A** and **38B,** wherein the transducer **38A** is a transmitter for transmitting an ultrasound wave **58,** and the transducer **38B** is the receiver, for receiving an ultrasound echo 60, from the interface **22** within the tissue **12.** In this manner, instrumentation dead time is reduced.

Preferably, the ultrasound sensor **38** is preset for a focal distance of about 5 mm, which is the desired depth **25** of the clean margin **24,** thus providing the most accurate results for this distance.

**Figure 3** further illustrates the structure **45** of the coaxial cable **44** and the tissue-type sensor **33.** Additionally, the position-tracking device **50** is shown. When correlated with a tissue coordinate system **54,** illustrated hereinbelow, in conjunction with Figure 6, it may be used together with the ultrasound sensor **38,** to provide a three-dimensional image of the tissue **12** and the abnormal tissue type **16** within.

The cable **46** carries the measurements to the computerized system **95,** described hereinbelow in conjunction with Figure 6.

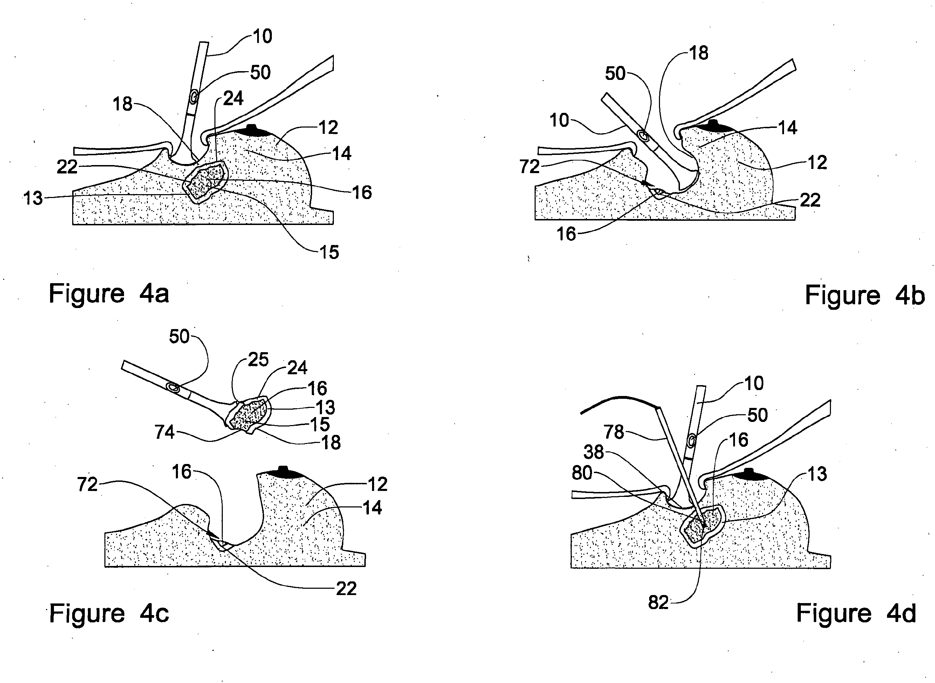

Referring further to the drawings, **Figures 4a-4d** further illustrate the operational manner of the integrated tool **10** for clean-margin assessment, in accordance with the present invention.

Generally, to localize the tumor within the breast, a radiologist may place a guide wire under x-ray or ultrasound guidance, so that the proximal tip of the guide wire, with respect to the tissue, is in the tumor. Alternatively, an imaging modality alone, for example, mammography, CT, ultrasound, or another imaging modality may be used to locate the tumor. The patient is then transported to the operating room, where the surgeon uses the guide wire, or the image, or palpation to locate the tumor in the breast and to excise a portion of tissue including the cancerous portion and a layer of healthy tissue surrounding the cancerous portion. The process of inserting a guide wire is termed, pre-procedure.

In accordance with the present invention, two methods are possible, without pre-procedure, as illustrated in **Figures 4a** - **4c****,** and with pre-procedure, as illustrated in **Figure 4d****.**

Thus, **Figures 4a** - **4c** schematically illustrate the use of the integrated tool **10** when no guide wire is used.

As seen in **Figure 4a****,** the integrated tool **10** may be used on the tissue surface **18,** during the removal of the portion **15,** to verify that the cutting proceeds as planned. At this stage, the near zone volume of the surface **18** should detected by the tissue type sensor **33** to be of the first tissue type **14** of healthy tissue, and the interface 22 with the second tissue type **16** should be detected at the desired depth **25.** Corrections can be made in real time.

As seen in **Figure 4b****,** the integrated tool **10** may be used on the tissue surface **18,** after the removal of the portion **15,** to verify that the all the cancerous tissue has been eliminated. At this stage, the near zone volume of the surface **18** should detected by the tissue type sensor **33** to be of the first tissue type **14** of healthy tissue, and no interface **22** and no second tissue type **16** should be detected. As seen in **Figure 4b****,** where a portion **72** of the second tissue type **16** remained, the integrated tool **10** will identify it both by the character of the near zone volume of the tissue surface 18 around the portion 72, and by the presence of the interface **22,** in back of the second tissue type **16,** indicating that two types of tissue remained.

As seen in **Figure 4c**, the integrated tool **10** may be used on the tissue surface **18,** of the removed portion **15,** after removal. This, to verify that the all the cancerous tissue is surrounded by the clean margin **24** of the first tissue type **14** of healthy tissue, and of sufficient depth **25.** At this stage, the near zone volume of the surface **18** should be of the first tissue type **14,** and the interface **22** should be detected at the desired depth **25.**

Additionally, as seen in **Figure 4c****,** where there is no clean margin, as shown by a surface **74,** the integrated tool **10** will identify it both by the character of the near zone volume of the tissue surface **18** at the surface **74,** and by the absence of the interface **22,** around the desired depth **25.**

**Figures 4d** schematically illustrates the use of the integrated tool **10** with a guide wire **78** that has been inserted during pre-procedure, with the help of x-ray or another imaging modality. This procedure often applies to non-palpable tumors, which are difficult to detect.

Preferably, the distance-measuring sensor **38** is an ultrasound transducer, and the guide wire **78** is visible by the ultrasound. Additionally, a guide-wire transducer **82** may be mounted on the tip **80,** for sending signals that may be received by the distance-measuring sensor **38.** Thus, the distance-measuring sensor **38** may estimate the distance to the tip **80,** hence the distance to the second tissue type **16.**

The guide wire transducer **82** may be, for example, a micro-electromechanical system (MEMS) ultrasound transducer, with a typical size of about 100 µm in diameter. Furthermore, the distance-measuring sensor 38 may include three transducers, for calculating the exact position of the guide wire transducer **82,** by triangulation. It will be appreciated that in the calculation of the distance between the guide wire transducer **82** and the distance-measuring sensor **38,** it is assumed that the sound velocity in cancerous tissue and in healthy tissue is about the same.

Alternatively, the sensor **82** at the tip **80** of the guide wire **78** may be a magnetic positioning device, coupled with an RF transmitter, for transmitting its position, via RF signals, which may be received by an RF receiver on the integrated tool **10.**

When the portion **15** has been removed, Figures 4b and 4c apply, as before.

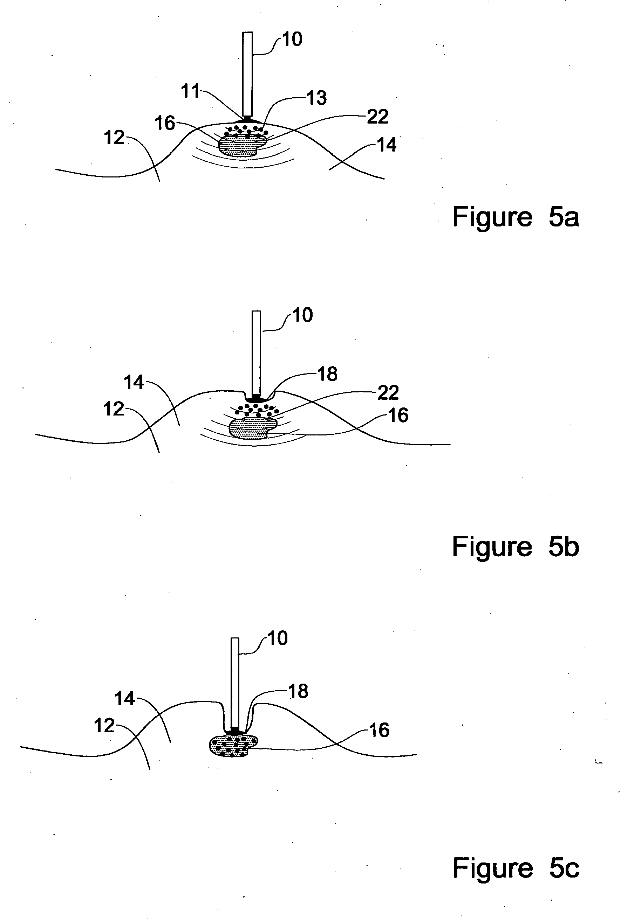

Referring further to the drawings, **Figures 5a** - **5c** further illustrate the operational manner of the integrated tool **10** for clean-margin assessment, in accordance with the present invention.

As seen in **Figure 5a****,** as a first step, the integrated tool **10** is applied to an external surface **11,** such as a skin, forming the surface **18,** prior to cutting and prior to the removal of the portion **15** (Figure 1). Alternatively, the surface **18** may be a lumen. The tissue-type sensor **33** will probably detect that the surface **18** is of the first tissue type **14** of healthy tissue, and the distance-measuring sensor **38** will detect the interface **22** with the second tissue type **16** at some depth.

As seen in **Figure 5b****,** when the incision begins, for the removal of the portion **15** (Figure 1), the integrated tool **10** is applied to the tissue surface **18,** now the tissue surface **18,** to verify that the cutting proceeds as planned. At this stage, the tissue-type sensor **33** will detect that the near zone volume of the tissue surface **18** is of the first tissue type **14** of healthy tissue, and the distance-measuring sensor **38** will detect the interface **22** with the second tissue type **16** at some depth, approaching the desired depth **25** of the clean margin **24.** Corrections and adjustments can be made in real time.

As seen in **Figure 5c**, if cutting went too far, the tissue-type sensor **33** will detect that the near zone volume of the tissue surface **18** is of the second tissue type **16** of abnormal tissue, and the distance-measuring sensor **38** will not be able to provide useful information, as no clean margin exists.

Referring further to the drawings, **Figure 6** schematically illustrates an overall computerized system **95,** for clean-margin assessment, in accordance with the present invention.

System **95** includes the tissue characterizing device, e.g. configure as the above described integrated tool **10,** having the structure **45,** on which the tissue-type sensor **33** and the distance-measuring sensor **38** are mounted. Preferably, both sensors are located at the proximal end **30,** with respect to the tissue. Additionally, the device **10** may include the position-tracking device 50, for providing its coordinates with respect to the frame of reference **54,** which defines a six-degree coordinate system, of x, y, z, and the rotational angles around them, ω, θ, and p.

Data from the device **10** is carried to appropriate analyzers, preferably associated with a computer **90** for analysis. It will be appreciated that the computer **90** may be a personal computer, a laptop, a palmtop, a microcomputer, or another computer, as known.

For example, where the tissue-type sensor **33** is an electrical properties sensor, constructed essentially as the coaxial cable **44** (Figures 2a - 2c), an electrical properties sensing module 94 includes, for example, an impedance analyzing external unit, such as Agilent 4396A, and a test fixture **89** connected via a coaxial cable to the impedance analyzing external unit.

Similarly, the distance-measuring sensor **38,** such as the ultrasound transducer **38** is associated with an ultrasound signal generator and analyzer **96.** The position-tracking device **50** may be associated with an analyzer **98.** The sensors may be battery operated or associated with power supply units.

The computer **90** which receives the data from the analyzers, preferably includes a user interface, for example, a keyboard **97,** or knobs, and may further include storage systems, such as a read and write drive **91,** a USB port **93,** and a display screen **92.**

It will be appreciated that where a different tissue-type sensor **33** is used, the unit **94** type will complement that sensor **33.** For example, where sensor **33** is an optical sensor, the unit **94** will be an optical analyzer. Similarly, where a different distance measuring sensor **38** is used, the unit **96** will complement that sensor **38.**

Information from the distance-measuring sensor **38** together with that of the position-tracking device **50** may be used for reconstructing a three-dimensional image of the tissue, by the computer **90.** Additionally, the three-dimensional image may be displayed on the screen **92.**

The system **95** may further include a guide wire **78.** At the proximal end **80,** the guide wire may include a sensor **82,** which may be an ultrasound transducer or a magnetic positioning device, coupled with a transmitter, for transmitting the positioning of the proximal tip, when inserted in the tissue, as taught hereinabove, in conjunction with Figure 4d. Preferably, the sensor **82** is wireless, and operates via external interrogation, for example, from the distance-measuring sensor **38,** or on battery.

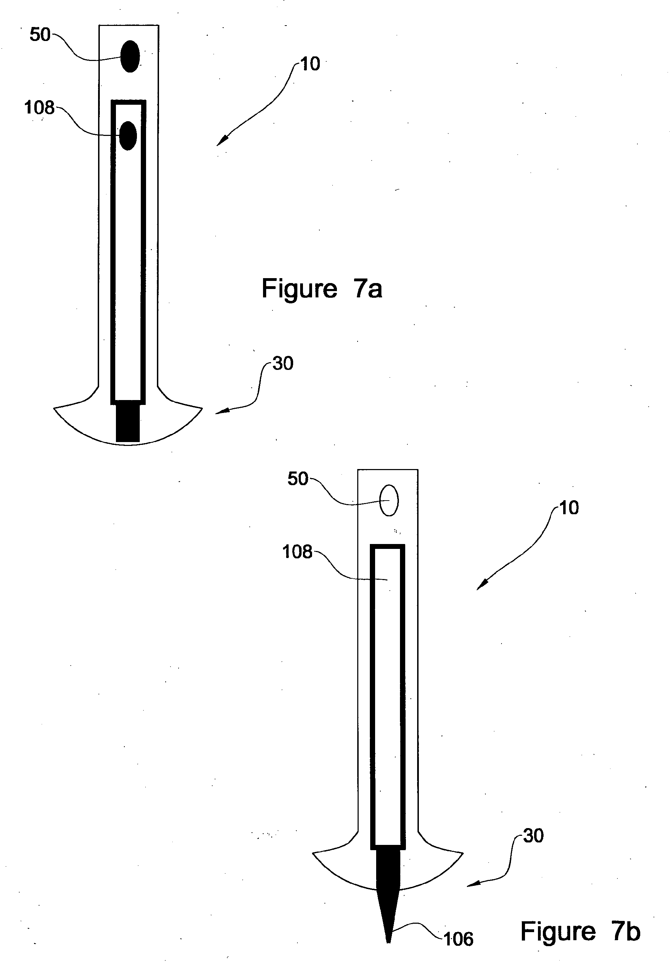

Referring further to the drawings, **Figures 7a** - **7d** schematically illustrate the tissue characterizing device **10,** which further includes a retractable knife **106,** in accordance with a preferred embodiment of the present invention.

As seen in **Figure 7a****,** the knife is retracted, and the tool is used as described hereinabove.

As seen in **Figure 7b****,** the knife is deployed, and the tool is used for removing the portion **15.**

Thus the surgeon may use the integrated tool **10** both for measuring and characterizing the clean margin and for removing the portion **15.**

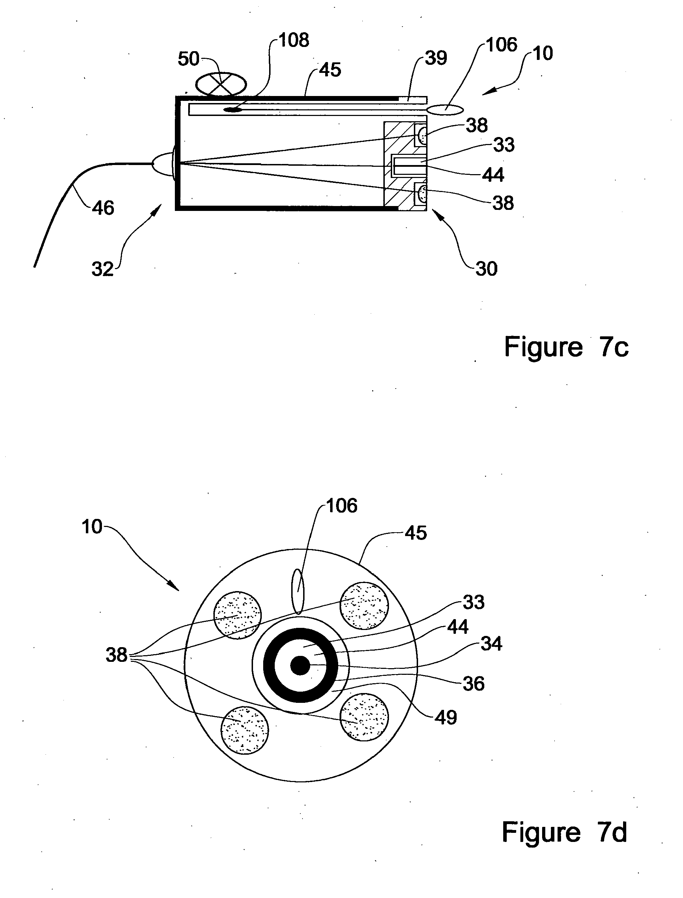

**Figure 7c** illustrates the proximal view of the integrated tool **10,** in accordance with the present embodiment, while **Figure 7d** provides a cross-sectional view.

Retraction and deployment are controlled by a knob **108.**

The knife **106** may be a cold knife, a diathermal knife, or another knife, as known.

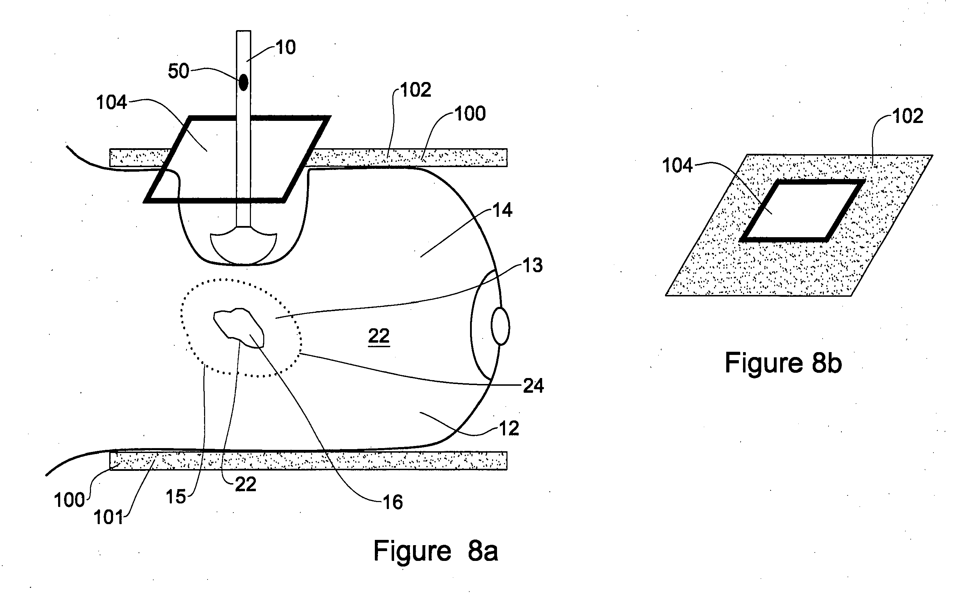

Referring further to the drawings, **Figures 8a** - **8b** schematically illustrate the device **10,** operative with a frame **100** for fixing the soft tissue **12,** in accordance with a preferred embodiment of the present invention.

The frame **100** has a support plate **101** and a compression plate **102.** The compression plate **102** defines an opening **104,** through which the integrated tool **10** may be inserted.

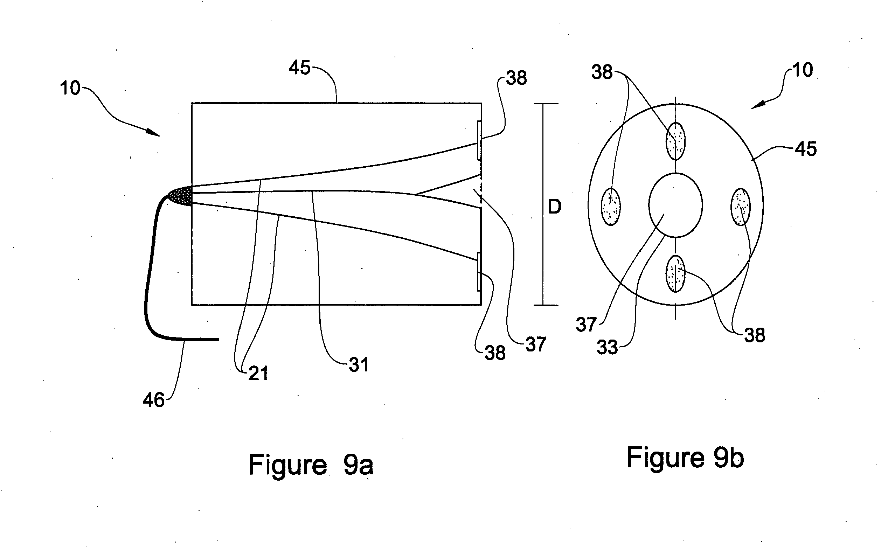

In accordance with the present invention various sensors may be used for the tissue-type sensor **33,** for characterizing the near zone volume of the tissue surface **18** in contact with the integrated tool **10.** These are illustrated below, in conjunction with Figures 9a - 12b.

Referring further to the drawings, **Figures 9a and 9b** schematically illustrate the integrated tool 10, wherein the tissue-type sensor **33** is formed as an RF or MW horn antenna **37,** mounted on the structure **45,** in accordance with still another embodiment of the present invention.

The RF or MW horn antenna 37 is associated with an RF/MW transmission line or wave guide **31,** while unit **94** (Figure 6) is an RF/MW generation, collection and analysis unit.

The present embodiment relies on RF microwave characterization by the generation of propagating radiation in the RF microwave region of the electromagnetic spectrum, towards the tissue, and measuring its reflection. The radiation is usually transmitted and received by an antenna, for example the horn antenna **37.** The tissue characterization is done by analyzing the amplitude and phase difference between the original waves to the reflected wave.

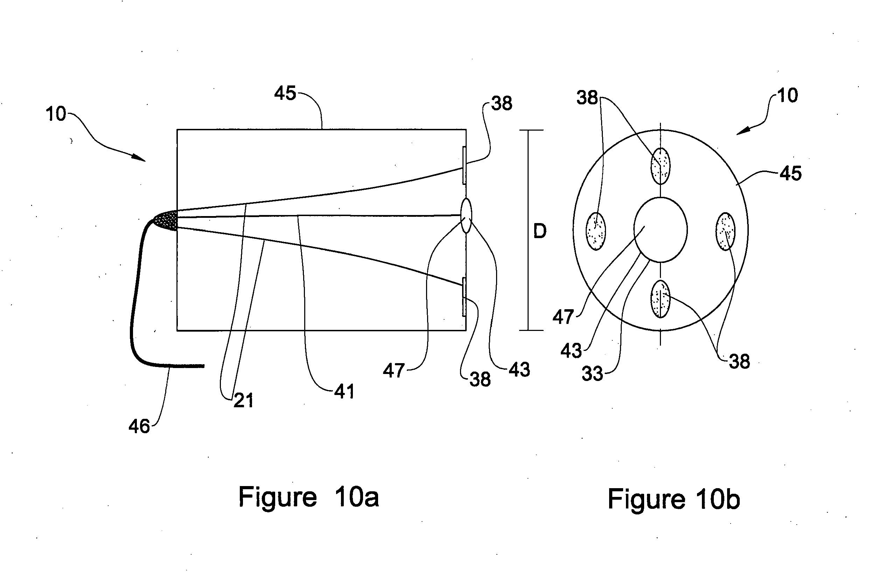

Referring further to the drawings, **Figures 10a and 10b** schematically illustrate the tissue characterizing (and locating) device **10,** wherein the tissue-type sensor **33** is formed as an optical sensor **47,** mounted on the structure **45,** in accordance with yet another embodiment of the present invention.

An optical signal is generated in an external unit, such as unit **94** (Figure 6) and transmitted via an optical fiber **41** to the tissue. The reflection of the light is then received in a dedicated module inside the optical unit. The optical energy is usually transmitted to and from the tissue via a lens **43.**

The details of optical signal generation, receiving and analyzing depend on the specific optical method that is chosen. For example, for reflection spectroscopy, tissue characterization relies on measuring the relative amplitude and phase of the reflected light versus the generated light. An example for the reflection spectroscopy method is described in commonly owned US Patent Application 10/298196, whose disclosure is incorporated herein by reference. It will be appreciated that other methods may be used, as known.

Alternatively, auto florescence may be used, for measuring emitted radiation, from the tissue, at different a wavelength than that originally transmitted. The emitted radiation occurs in response to excitation by impinging radiation, and may be used for tissue characterization, for example, as used by Xillix Technologies Corp., #100-13775 Commerce Parkway, Richmond, British Columbia, Canada V6V 2V4, Telephone: 604-278-5000, and described in http://www.xillix.com/index home.cfm. It will be appreciated that other methods may be used, as known.

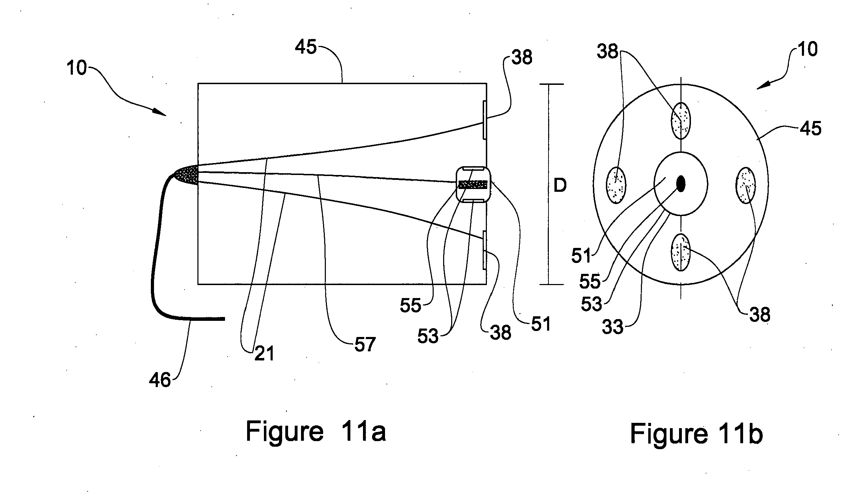

Referring further to the drawings, **Figures 11a and 11b** schematically illustrate the device for clean-margin assessment, wherein the tissue-type sensor **33** is formed as an MRI sensor **51,** in accordance with yet another embodiment of the present invention.

The MRI sensor **51** has a permanent magnet **55,** enclosed in an RF coil **53,** for example, as taught in commonly owned US Patent Application 2005/0021019 to Hashimshony et al., entitled "Method and apparatus for examining substance, particularly tissue, to characterize its type," whose disclosure is incorporated herein by reference, and in U.S. Patent 5,572,132, to Pulyer, et al., entitled, "MRI probe for external imaging," whose disclosure is incorporated herein by reference.

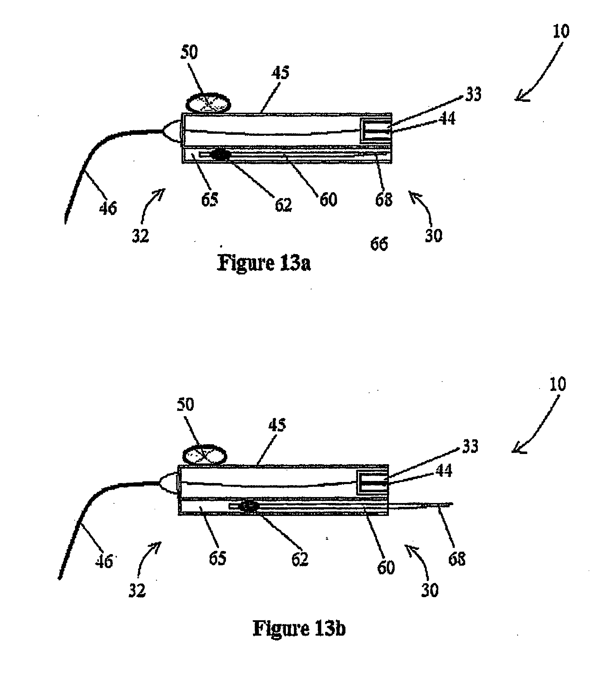

In accordance with the present invention various sensors may be used for the distance-measuring sensor **38,** as illustrated below, in conjunction with Figure 13.

It will be appreciated that many other tissue characterization sensors may be used, as known. These may include a sensor for tissue electromagnetic properties, a dielectric sensor, an impedance sensor, a sensor for optical fluorescence spectroscopy, a sensor for optical reflectance spectroscopy, an MRI sensor, a temperature sensor, and infrared thermography sensor, or another tissue-characterization sensor, as known.

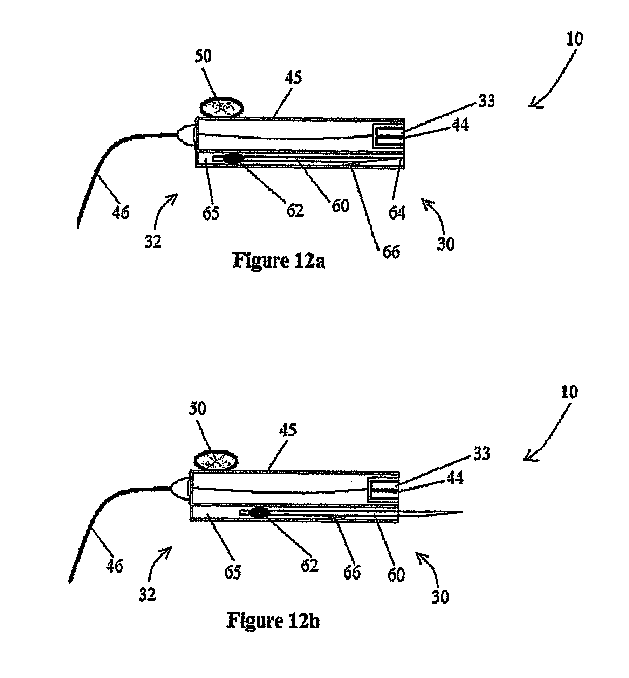

Referring further to the drawings, **Figures 12a and 12b** schematically illustrate the tissue characterizing device **10,** wherein the distance-measuring sensor **38** is formed as a strain gauge **66,** in accordance with still another embodiment of the present invention.

The present embodiment utilizes the approach of US Patent 6546787 to Schiller et al., whose disclosure is incorporated herein by reference, and which provides an apparatus and method for detecting a distance from a tissue edge to a malignant tissue, enclosed therein, i.e., a margin. The apparatus comprises a needle having a strain gage, mounted on one of the needles walls. Strain signals are collected as the needle is moved through the tissue. The needle is inserted at different points to allow data collection from different points within the tissue. The data is sent together with its spatial coordinates to a computerized system, which provides an image of the structure of the examined tissue.

As seen in **Figures 12a and 12b****,** the structure **45** of the device **10** may include a lumen **65,** wherein a needle **60** may be retracted and deployed, via a knob **62.** The needle has a sharp edge **64,** for penetrating the tissue. The strain gauge **66** senses the tissue resistance to the penetration, and provides data of resistance as a function of needle penetration depth. These measurements may be performed at various locations along the tissue surface **18.**

Referring further to the drawings, **Figures 13a and 13b** schematically illustrate the device **10,** wherein the distance-measuring sensor **38** is formed as a pressure sensor **68,** at the needle's tip, in accordance with yet another embodiment of the present invention.

Again, the structure **45** of the device **10** may include the lumen **65,** wherein the needle **60** may be retracted and deployed, via the knob **62.** The pressure sensor **68** senses the tissue resistance to the penetration, and provides data of resistance as a function of needle penetration depth. These measurements may be performed at various locations along the tissue surface **18.**

It will be appreciated that a non-invasive imager may be used for the distance-measuring sensor **38,** for example, an MRI sensor.

Accordingly, the device **10** may be formed, for example, with the tissue-type sensor **33** being an optical sensor, and the distance-measuring sensor **38** being an on-invasive imager, such as an MRI sensor.

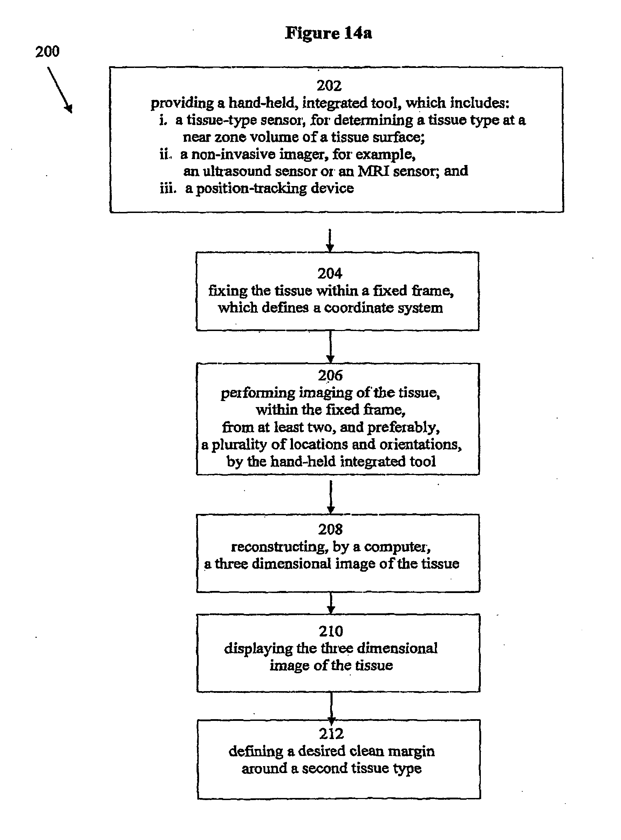

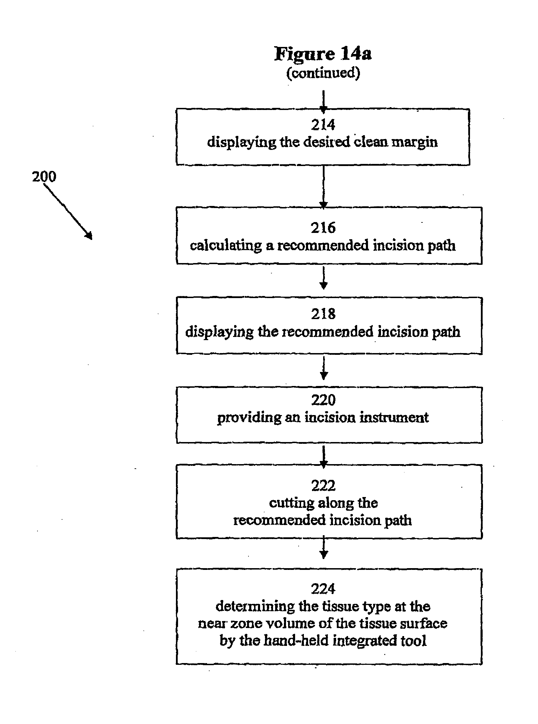

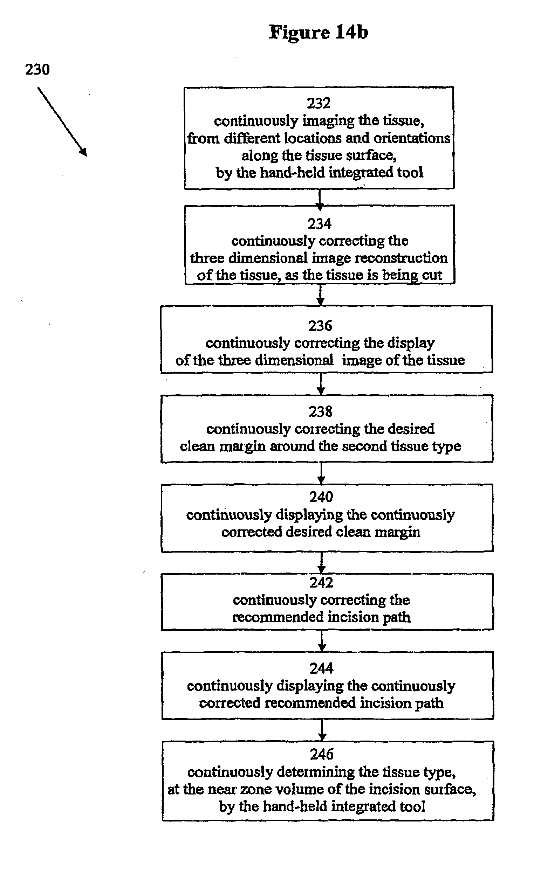

Referring further to the drawings, **Figures 14a** **and** **14b** illustrate, in flowchart forms, surgical methods of tumor removal, using the integrated tool 10, in accordance with embodiments of the present invention,

As illustrated in **Figure 14a****,** a method **200** provides a computer-guided surgery, as follows:
in a box **202:** providing the hand-held, integrated tool 10, which includes:
   1. the tissue-type sensor **33,** for determining a tissue type at a near zone volume of a tissue surface;
   2. the non-invasive imager **38,** for example, an ultrasound sensor, or an MRI sensor; and
   3. the position tracking device **50.**
in a box **204:** fixing the tissue within a fixed frame, which defines a coordinate system, preferably of six-degrees, x, y, z, and the rotational angles around them, ω, θ, and p.
in a box **206:** imaging the tissue, within the fixed frame, from at least two, and preferably, a plurality of locations and orientations, by the hand-held device **10.**
in a box **208:** reconstructing, by a computer, a three dimensional image of the tissue.
in a box **210:** displaying the three dimensional image of the tissue.
in a box **212:** defining a desired clean margin around a second tissue type.
in a box **214:** displaying the desired clean margin.
in a box **216:** calculating a recommended incision path.
in a box **218:** displaying the recommended incision path.
in a box **220:** providing an incision instrument.
in a box **222:** cutting along the recommended incision path.
in a box **224:** determining the tissue type at the near zone volume of the tissue surface, by the hand-held device **10.**

As illustrated in **Figure 14b****,** a method **230** further provides continuous correction to the method **200,** as follows:
in a box **232:** continuously imaging the tissue, from different locations and orientations along the tissue surface, by the hand-held device **10.**
in a box **234:** continuously correcting the three dimensional image reconstruction of the tissue, as the tissue is being cut.
in a box **236:** continuously correcting the display of the three dimensional image of the tissue.
in a box **238:** continuously correcting the desired clean margin around the second tissue type.
in a box **240:** continuously displaying the continuously corrected desired clean margin.
in a box **242:** continuously correcting the recommended incision path.
in a box **244:** continuously displaying the continuously corrected recommended incision path.
in a box **246** continuously determining the tissue type, at the near zone volume of the incision surface, by the hand-held device **10.**

Preferably, the knife is integrated with the tool, as taught in conjunction with Figures 7a - 7d.

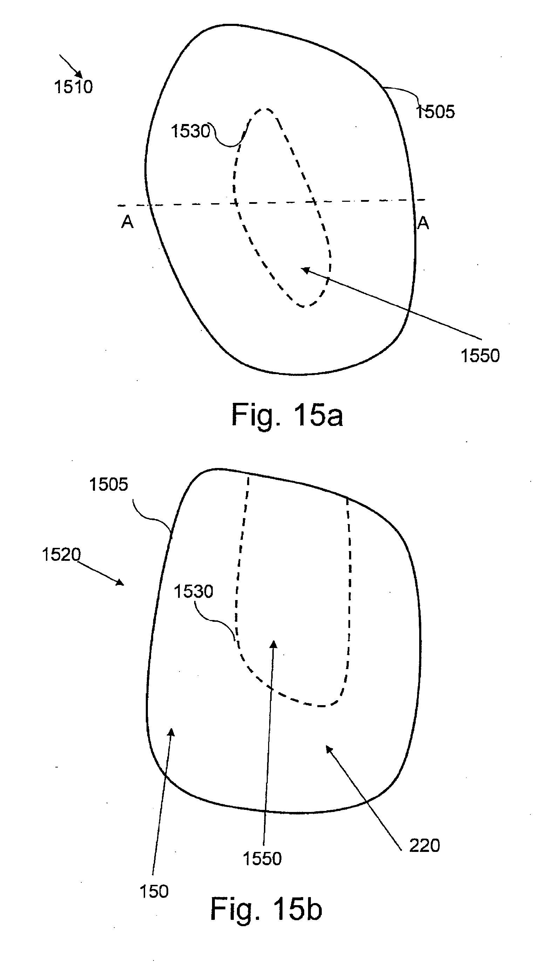

Referring further to the drawings, **Figures 15a - 15c** schematically illustrate the principles of providing a clean margin, in accordance with the present invention.

Figures 15a and 15b illustrate a top view **1510** and a cross sectional view **1520,** respectively, of a tissue part **1505** and an incision surface contour **1530** within tissue part **1505.** The incision surface **1530** surrounds a tissue region (portion) **1550** which may be removed from the tissue part **1505** during a surgical process for achieving a clean margin (i.e. obtaining the incision surface **1530** or the near zone at the incision surface **1530** with no abnormal tissue cells). In other words, identification of a clean margin within the surrounding or periphery of the tissue portion **1550,** which has abnormal tissue cells signifies that tissue **1550** can be removed and no removal of additional surrounding tissues is required.

The tissue portion **1550** may include for example a lesion, or a tumor, or any other abnormal tissue. The lesion, or the tumor, or the abnormal tissue, is to be fully and completely removed, with a clean margin surrounding it. The tissue part **1505** and the tissue portion **1550** therein may be a skin portion, a body lumen portion, an organ, an anatomical feature, some portion of intra-corporeal tissue, or a combination theirs. The tissue **1550** may be removed from a body, either completely or partially. The incision surface **1530** may be an incision surface contouring an organ, or an anatomical feature. According to some embodiments of the present invention the incision surface **1530** may be defined by a diagnostic modality, or by a surgeon.

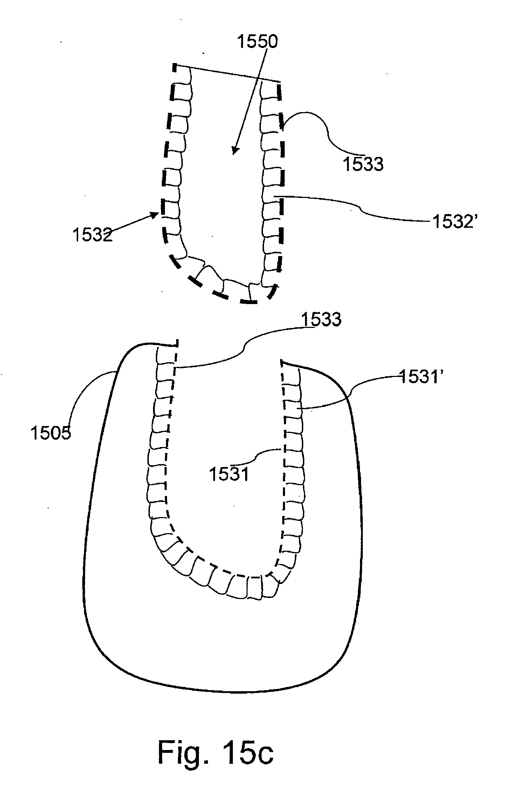

As shown in **Figure 15c****,** the excision of tissue portion **1550** from the tissue **1505** (along the incision contour **1530)** forms two separated surfaces (illustrated by a dashed curve **1533).** A first surface, e.g. surface **1531,** includes newly exposed surface segments **1531'** of the tissue **1505.** The surface **1531** may include the inner surface, the intact tissue related surface, the cavity surface. A second surface, e.g. surface **1532,** includes newly exposed surface segments **1532'** of the tissue portion **1550.** The surface **1532** may also be termed as the outer surface, the removed tissue related surface, the excised tissue related surface, the lump surface.

A process for achieving a clean margin includes a characterization process followed by an incision/additional tissue removal process. According to some embodiments of the present invention, during the characterization process the inner surface **1531** and/or the outer surface **1532** are characterized to determine whether a tissue portion such as the tissue portion **1550** has been excised with the clean margins. The incision/additional tissue removal process follows the characterization process. The characterization and incision/additional tissue removal cycle may be continued until for example the characterized tissue surface contains no cancerous cells, and thus has a clean margin. Alternatively, the incision/additional tissue removal process following the characterization process may include removal for example of a specific organ, or anatomical feature, for example without additional characterization cycles. According to some embodiments of the present invention, during or following the incision/additional tissue removal process an additional characterization process and/or corrections for the clean margins may be preformed.

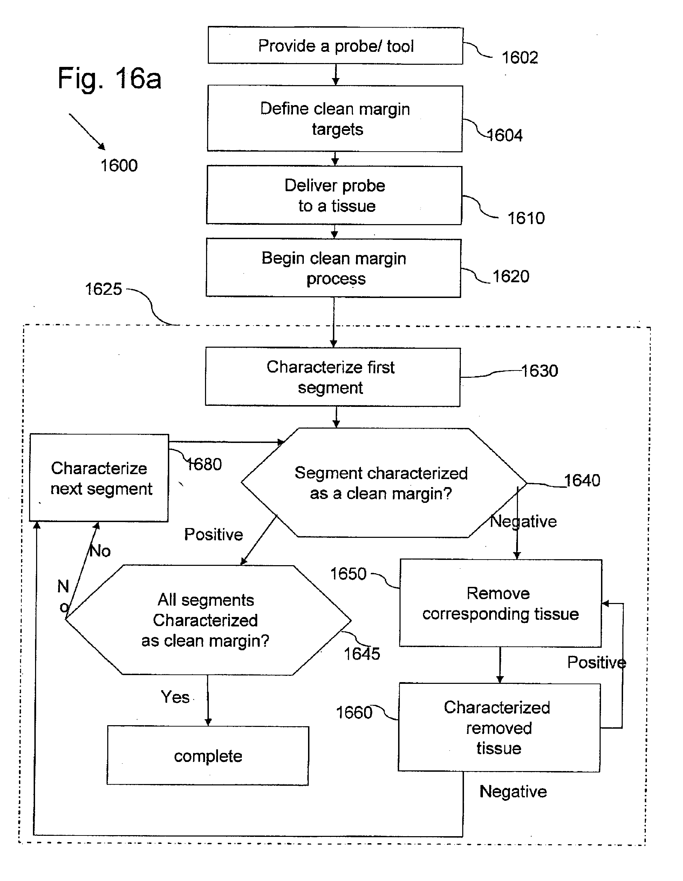

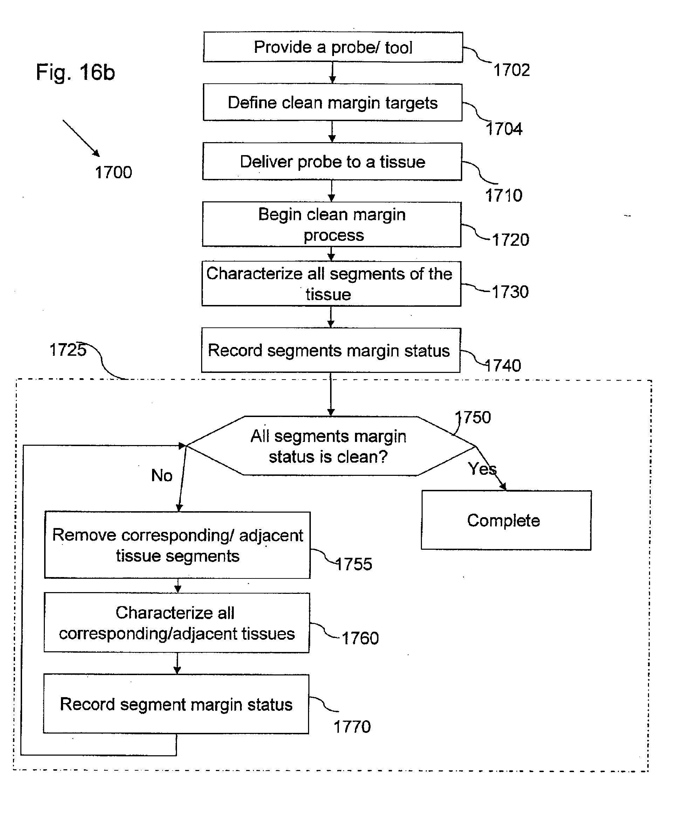

Reference is now made to **Figures 16a** **and** **16b** illustrating flowcharts **1600** and **1700** of a method for providing a clean margin of healthy tissue around a malignant tumor or abnormal tissue, in accordance with some embodiments of the present invention. As illustrated in flowchart **1600,** a medical probe or tool for characterizing a tissue is provided (step **1602).** The probe includes one or more tissue-type sensors, such as the above-described tissue-type sensor **33,** or an array of sensors for determining the characteristics of a tissue surface, such as tissue surfaces **1531** or **1532,** for example in the near zone volume of the tissue surface. According to some embodiments of the present invention, the probe is configured as the above-described integrated tool 10 for clean-margin assessment. According to some other embodiments of the present invention, the probe is configured similar to that described in US Application No. 10/891,750 and/or in U.S. Pat. No. 6,813,515, each of which are assigned to the common assignee of the present application and each of which is hereby incorporated by reference. It should be understood that the probe may have other configurations and other sets of components.

The clean margin status targets are defined (step **1604).** To this end, the clean margin status targets may be selected from, but is not limited to: no abnormal tissue at the characterized tissue surface; no abnormal tissue up to a given depth from the characterized tissue surface, such as a 1 mm or 2mm depth, or up to 20 mm depth. The probe is delivered to a tissue (step **1610),** such as the tissue **1505** shown in Figures 15a-15c. The clean margin process achievement begins (step **1620),** and the process enters a control cycle (steps **1625).** A first segment is characterized (step 1630), for example at a 'near zone' tissue surface (e.g. 0-20mm), to determine, preferably in real-time, based upon the characterization of the tissue at the present segment, whether the first segment is characterized as having a clean margin (step 1640). The first segment may be for example one of the tissue segments 1531' or 1532'. A characterization result/data or a margin status of each examined segment may be recorded in a memory utility located for example in the probe, and may be further displayed on the computer screen. The margin status of each examined segment may be further transmitted to the external computer or to an external memory device such as a removable memory e.g. a DiskonKey or other small and portable memory device. The margin status of each segment which was recorded or saved may be used for example for additional procedures such as pathology procedure related to the examined tissue e.g. tissue **1505** or to a different body lumen or anatomical feature of a patient.

According to some embodiments of the present invention, a session data may be saved for example in a computerized system, such as the above described computerized system **95,** for further analysis. The session data may include for example, a reconstructed three-dimensional image of a tissue portion (e.g. an examined tissue surface such as the tissue portion **1550),** the coordinates and margin status of all segments in the tissue portion. The session data may be exported and transmitted to an external device/computer or to an external memory device such as a removable memory e.g. a DiskonKey or other small and portable memory device. The session data which was recorded or saved may be used, for example, in additional procedures, such as pathology procedures, related to the examined tissue, additional surgical or diagnostic procedures, related to the respective patient.

A segment margin status result may be defined as a positive result or negative result. If positive, e.g. the first segment is characterized as having a clean margin, then the probe is displaced and relocated to the next tissue segment to determine whether all segments of the tissue surface were characterized as having a clean margin (steps **1645, 1680, 1640).** The relocation of the probe may be manual, semi-manual or automatic employing for example, a two-dimensional or three-dimensional computer controlled stage, as is known in the art. There may be a computer program which controls the stage and defines the sequence of moving the probe from one location or segment to the next. In cases, where the relocation is manual or semi-manual, the system may provide an operator with specific instructions on how and to whereto move the probe. If all the tissue segments are characterized as having a clean margin, then the achieving clean margin process is completed. If no, the probe is displaced and relocated to the next tissue segment to characterize the next tissue segment (step **1680)** and determine whether the next tissue segment has a clean margin or not (step **1640).** The next segment may be located, for example adjacent to or in proximity to the previously characterized segment. If the first characterized segment has no clean margin, then a tissue segment adjacent to the first tissue segment is removed from the body (step **1650).** The tissue removal may be done using an incision instrument, which may for example be attached to the probe to enable cutting and removing the tissue region while characterizing the respective tissue segment. The removed tissue segment or the surface of the tissue from where the tissue was removed is characterized (step **1660)** for determining whether it has a clean margin. If negative, e.g. the removed tissue segment or the surface of the tissue from where the tissue was removed includes a clean margin, then the clean margin assessment process is continued and the process returns to step **1680** for characterizing the next segment. If positive, e.g. the removed tissue segment or the surface of the tissue from where the tissue segment was removed does not include a clean margin, then the process returns to step **1650** and the tissue adjacent to at least the location of the tissue segment at which there was no clean margin is removed from the body.

According to some embodiments of the present invention the output data relating to the margin status of for example all characterized tissue segments may be recorded and transmitted to the computer system and may be displayed on the computer screen. According to some embodiments of the present invention the clean margin process may be performed for providing a clean margin during a procedure for characterizing an anatomical feature. An example of anatomical feature may be a prostate.

**Figure 16b** shows flowchart **1700** according to another example of the present invention. A probe or tool for characterizing a tissue is provided (step **1702),** the probe/tool may include one or more tissue-type sensors, such as the above described tissue-type sensor **33,** for determining the characteristics of a tissue surface, such as tissue surfaces **1531** or **1532,** for example in the near zone volume of the tissue surface. The probe may be configured as the above described integrated tool 10 for clean-margin assessment, or may have other configurations and other sets of components.

The clean margin process targets are defined (step **1704).** The clean margin process targets may be selected, as for example but is not limited to: no abnormal tissue at the characterized tissue surface; no abnormal tissue up to a given depth from the characterized tissue surface, such as a 1 mm or 2mm depth , or up to 20 mm depth.. The probe is delivered to a tissue (step 1710), such as the tissue **1505,** or tissue portion **1550,** shown in Figures 15a-15c. The clean margin process begins (step **1720).** All the segments **(1531'** and or **1532')** of the tissue surface **1531** and or **1532** are characterized (step **1730).** The margin status of all segments is reordered (step **1740)** or registered for example by a computerized system, such as the computerized system **95** for clean-margin assessment, described hereinabove in conjunction with Figure 6. The margin status which is based on the defined clean margin targets may be negative, e.g. the segment is characterized as having a clean margin, or positive, e.g. the segment is characterized as not having a clean margin.

According to some embodiments of the present invention the margin status of all segments which was reordered or registered may be used for determining an incision path, for example for decision-making during an operation as to the delimitation of the abnormal tissue e.g. the segments which were characterized as not having a clean margin.

The achieving clean margin process enters a control cycle (steps **1725)** which includes the following: Based upon the margin status of each segment, it is determined, preferably in real time, whether all the tissue segments are characterized as having a clean margin (step **1750).** If yes, e.g. all the tissue segments are characterized as having a clean margin then the achieving of a clean margin process is completed. If no, then tissue segments which correspond and/or are adjacent to the location of the tissue segments at which there was no clean margin are removed (step **1755),** for example using a scalpel, or a diathermal knife. The removed tissue segments and/or the surface of the tissue from where the tissue was removed are characterized (step **1760),** and their margin status is recorded (step **1770).** Then, the clean margin process returns to the first step **1750** of the control cycle for determining whether the removed tissue and/or the surface of the tissue from where the tissue segments were removed are characterized as clean margin. The clean margin process is continued in the control cycle until the margin status of all the characterized tissue segments is clean.

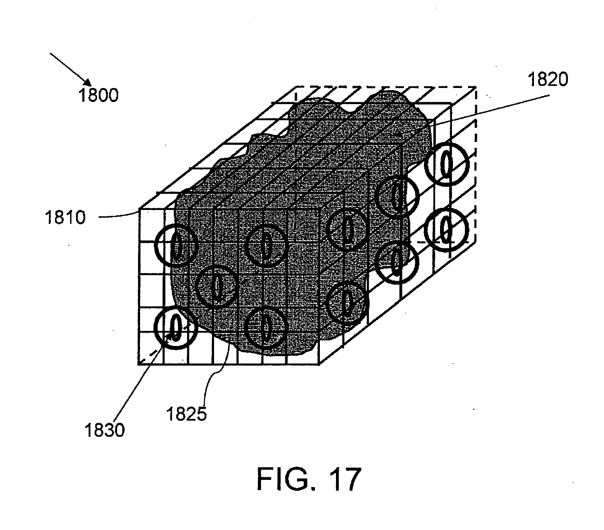

Reference is now made to **Figure 17** illustrating a device **1800** for holding and characterizing a tissue or an anatomical feature, for example during a clean-margin assessment process, in accordance with some embodiments of the present invention. The device **1800** includes a body or housing **1810,** configured for receiving and holding a tissue or the tissue portion (e.g. as shown in Figures 15a-15c), or a body lumen portion, a skin portion or an anatomical feature. According to one embodiment of the present invention, one or more sensors such as tissue-type sensors **1830,** or an array of sensors such as a rectangular array or matrix of optical sensor elements, may be attached or mounted on the housing **1810** for sensing and characterizing the tissue surface **1825,** for example of an anatomical structure **1820,** to indicate a clean margin.

The housing **1810** may be shaped to conform to the surface of the anatomical feature **1820.** Therefore, the anatomical feature **1820** may be sensed or scanned from any direction, without being limited by the shape of the housing **1810.** According to one embodiment of the present invention, the sensors or the array of sensors may be attached to the inner side of the housing **1810** for sensing or scanning for example an anatomical structure which is enclosed by the housing. According to another embodiment of the present invention, the sensors or the array of sensors may be attached to the outer side or surface of the housing **1810** for sensing or scanning for example an anatomical structure which surrounds the outer side of housing **1810.** According to some embodiments of the present invention, one or more sensors such as the as tissue-type sensors **1830** may be attached to and cover the whole surface of the housing **1810,** thus enabling sensing or scanning the whole surface of the anatomical structure simultaneity in real time.

The housing **1810** may be formed as a rigid body such as cube, or a sphere, or an ellipsoid. Additionally, or alternatively, the housing **1810** may be formed, for example as a flexible body such as a stretchable body, an expansible body, a sac-like mesh, a stretchable stocking, or a resilient cage.

Such housing **1810** may be similar to various embodiments described, for example, in international publication number WO 2006/092797, entitled "Device And Method For Transporting And Handling Tissue", assigned to the common assignee of the present application and incorporated herein by reference.

According to some embodiments of the present invention, a tissue surface, such as the tissue surface **1825,** may be scanned or sensed by using a relative displacement between the housing and/or the sensors, e.g. by rotating the sensors **1830** or an array of sensors and/or the housing **1810,** using for example a robotic arm or a motor. For example, one or more sensors **1830** may be connected to a robotic arm which is configured to move and rotate the sensors **1830** and scan the tissue surface **1825** of the anatomical feature **1820** to indicate the margin status at the anatomical feature **1825,** while the housing **1810** holds the anatomical feature **1820.** According to some other embodiments of the present invention, the housing **1810** may be rotated as it holds the anatomical feature **1820** and the sensors **1830** or the array of sensors may sense and/or scan the anatomical feature **1820** to indicate the margin status at the anatomical feature **1825,** while the sensors or the array of sensors are stable and fixed.

The tissue surface **1825** may include a specific positional reference with respect to the body from which it was taken, or is being taken, and the device **1800** is designed to maintain the tissue positional reference, by providing, for example a rigid frame of reference for it.

The feature/tissue characterizing device **1800** may be applied to the feature/tissue after the tissue **1825** or the anatomical feature **1820** have been removed from the body, or while the tissue **1825** or the anatomical feature are being removed.

In operation, a tissue (such as the tissue **1505** or the tissue portion **1550** shown in Figures 15a-15c), a body lumen portion, a skin portion or an anatomical feature, such as the anatomical feature **1820** may be inserted into the device **1800,** or may be attached to the outer surface of the housing **1810** for identifying whether there is a clean margin, for example at the tissue surface of the anatomical feature. The tissue or the anatomical feature **1820** is characterized, preferably in real time, by rotating the housing **1810** and/or the sensors **1830** of the device **1800** or by activating the array of sensors. Signals from the sensors **1830** are transferred for analysis to a computerized system, such as the computerized system **95** for clean-margin assessment, described hereinabove in conjunction with Figure 6. If the anatomical feature **1820** is characterized as having a clean margin then the clean margin process is completed. If the anatomical feature does not have a clean margin then an additional anatomical feature adjacent to the anatomical feature which did not have a clean margin, or a tissue corresponding/adjacent to at least the location of the tissue at which there was no clean margin, is removed from the body as described hereinabove in conjunction with Figures 16a and 16b.

According to some embodiments of the present invention, the device **1800** may be constructed as a continuous surface carrying sensors, such as sensors **1830,** mounted on its inner side. Such a continuous surface has an opening for connection to a vacuum source. The device **1800** may further include a mechanism for generating suction within it, for example by connecting it to a vacuum source. When suction (e.g. vacuum) is applied to the housing **1810** of device **1800,** for example when a tissue portion is enclosed within the housing **1800,** negative pressure present within the housing **1810** will result in the deformation of the housing so that its shape will match to the tissue within it, thereby attaching the tissue or the anatomical feature **1820** to the sensors **1830** or to the active areas thereof. Once the tissue within the housing has been attached to the sensor array active areas, a tissue characterization process is initiated.

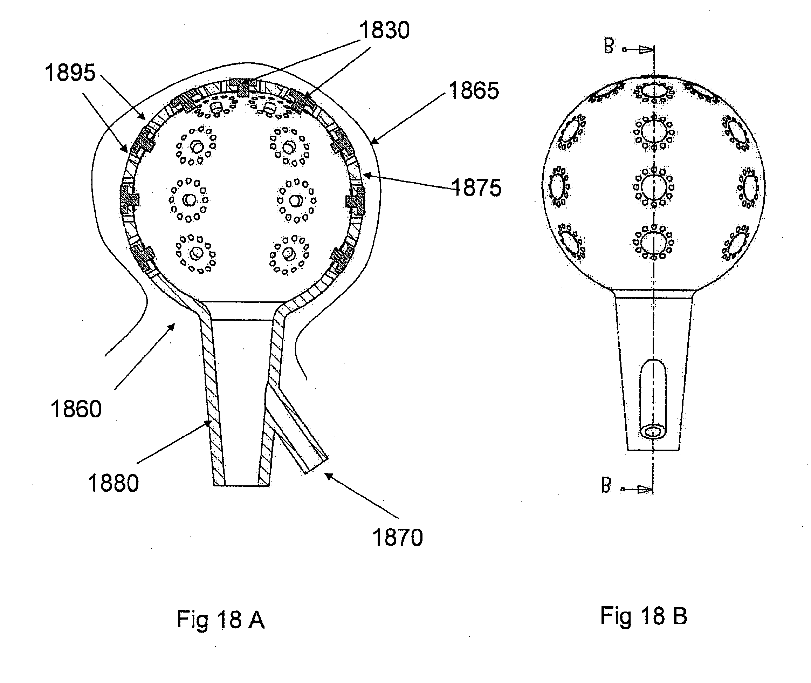

According to some embodiments of the present invention, as shown in **Fig. 17** and more specifically in **Figs. 18A and 18B****,** one or more sensors such as sensors **1850** or **1830,** e.g. array sensors, may be mounted on or attached to the outer surface of a sensor array frame structure **1860** or the device **1800** to characterize tissue, such as tissue **1865** enclosing and/or surrounding the sensor array frame structure **1860** or device **1800.** The sensor array frame structure **1860** or device **1800** may be formed, for example, but not limited to, as a sphere, an ellipsoid, a tube, or a shape conforming to a body anatomical feature. The sensor array structure **1860** further includes a signal communication bundle **1870** for transmitting and receiving signals from and to the sensors **1850.** The signal communication with the sensors **1850** may be performed, for example as described in US 2008/0021343 which is assigned to the assignee of the present application and which is incorporated herein by reference. Each of the sensors may have a dedicated signal communication line. Alternatively, a common signal communication line is employed associated with multiple sensors and operable with multiplexing, for example, time multiplexing, or wavelength multiplexing.

The tissue **1865** surrounding the sensor array structure may be in a form of a closed continuous tissue surface, for example a cavity or a closed lumen. The cavity may be either a natural body cavity, or a cavity formed by removal of tissue during a surgical procedure, for example partial mastectomy or lumpectomy breast cancer removal surgical procedures. The closed lumen may be, for example, a blocked or truncated or an artificially closed lumen.

According to some embodiments of the present invention, the sensor array structure **1860** may include a mechanism for generating suction within it, configured to enable a contact and release between the sensor array frame structure **1860** and the tissue **1865,** ensuring a reliable and effective tissue-characterization.

As shown in the present example, the sensor array frame structure **1860** includes one or more connection locations (openings and possible appropriate connectors extending from said openings) **1880** for connecting a vacuum source (not shown) to the sensor array frame structure **1860,** and one or more perforations (holes) **1895,** arranged in a spaced-apart relationship within the surface of the structure **1860** to enable vacuum communication between the vacuum source and the outer surface **1875** of the structure **1860.** When the sensor array structure **1860** is surrounded by a closed continuous tissue surface, such as tissue **1865,** the holes **1895** enable vacuum communication between the vacuum source and the closed continuous tissue surface surrounding the sensor array structure.

In operation, for example during surgery, the sensor array frame structure **1860** is inserted, for example, into an opening in a closed continuous tissue surface. Following the insertion of the sensor array frame structure **1860,** the opening of the closed continuous tissue surface is tightly attached around the connection location **1880,** thus forming a closed continuous tissue surface with its only opening **1880** directly connected to the vacuum source. When vacuum (i.e. suction) from the vacuum source is applied to the interior of the sensor array structure, vacuum communication between the inner and outer surfaces of the sensor array structure and the closed continuous tissue surface is formed, due to the existence of holes 1895 across the structure surface. The negative pressure present at the outer surface of the sensor array structure **1860** results in the collapse of the closed continuous tissue surface **1865** and attachment of this surface onto the external surface of the sensor array structure **1860,** where the sensors **1850** are located. Once the closed continuous tissue surface has attached itself to the sensor array active areas, a tissue characterization process is initiated.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination.

Although the invention has been described in conjunction with specific embodiments thereof, it is evident that many alternatives, modifications and variations will be apparent to those skilled in the art. Accordingly, it is intended to embrace all such alternatives, modifications and variations that fall within the spirit and broad scope of the appended claims. All publications, patents and patent applications mentioned in this specification are herein incorporated in their entirety by reference into the specification, to the same extent as if each individual publication, patent or patent application was specifically and individually indicated to be incorporated herein by reference. In addition, citation or identification of any reference in this application shall not be construed as an admission that such reference is available as prior art to the present invention.

## Claims

1. A device (1800) for use in monitoring a tissue (1825), the device (1800) being configured for holding and characterizing a tissue portion during the monitoring, said device (1800) comprising a housing (1810) and a tissue-type sensor (1830) unit mounted on the housing (1810), the device being **characterized in that** said housing (1810) has an inner surface and an outer surface and the tissue-type sensor (1830) unit is mounted on either one or both of said inner and outer surfaces, the housing (1810) having one of the following configurations for holding the tissue portion being monitored:
(1) the housing (1810) is configured for enclosing the tissue portion inserted into the housing, and carries the tissue-type sensor (1830) unit mounted on at least the inner surface thereof; or
(2) the housing (1810) carries the tissue-type sensor (1830) unit mounted on at least the outer surface of the housing (1810) having a portion thereof associated with an attachment mechanism for attaching the tissue portion to the outer surface of the housing by causing the tissue portion surrounding the outer surface of the housing (1810) to wrap said portion of the housing;
the device (1800) being thereby configured and operable for characterizing the tissue portion being held by the housing (1810).

2. A device (1800) according to claim 1, wherein the sensor unit comprises a plurality of sensor elements.

3. A device (1800) according to claim 2, wherein said sensor elements are distributed within the surface of the housing (1810).

4. A device (1800) according to claim 1 or 2, comprising a robotic arm for rotating said sensor unit with respect to the housing (1810).

5. A device (1800) according to claim 1 or 2, comprising a robotic arm for rotating the housing (1810) with respect to said sensor unit.

6. A device (1800) according to any one of the preceding claims, wherein the housing (1810) has a body having one of the following configurations: a rigid body, a flexible body, a stretchable body, and an expansible body.

7. A device (1800) according to claim 6, wherein the body of the housing (1810) has a shape of an anatomical feature.

8. A device (1800) according to any one of the preceding claims, comprising at least one signal communication bundle for transmitting and receiving signals from and to the sensor unit.

9. A device (1800) according to any one of the preceding claims, wherein the housing (1810) is configured for enclosing the tissue portion, the device comprising an attachment mechanism configured to attach the tissue portion to the inner surface of housing (1810).

10. A device (1800) according to any one of the preceding claims, wherein the attachment mechanism comprises a vacuum system.

11. A device (1800) according to claim 10, comprising a connector to a vacuum source.

12. A device (1800) according to claim 11, wherein said housing (1810) comprises one or more openings to enable vacuum communication between said tissue and said vacuum source.

13. A device (1800) according to any one of the preceding claims, configured for monitoring the tissue (1825) while in a subject's body, or for monitoring the removed tissue (1825) outside the subject's body.

14. A device (1800) according to claim 13, configured for monitoring the tissue (1825) during the tissue portion removal from a subject's body.

15. A device (1800) according to any one of the preceding claims, wherein the housing (1810) is configured to be surrounded by or to enclose the tissue portion being in the form of a cavity or a closed lumen.

16. A system for use in monitoring a tissue (1825), the system comprising:
- a device (1800) of any one of the preceding claims; and
- a computerized system (95) connectable to said device (1800), the computerized system (95) comprising:
o an analyzer utility configured and operable for analyzing data generated by the tissue-type sensor (1830) unit, determining whether a clean margin (24) of healthy tissue exists in a periphery region of the tissue portion, and generating data indicative of the analysis results; and
o an output device, which provides output data corresponding to said data generated by the analyzer utility.

## Patentansprüche

1. Vorrichtung (1800) zur Verwendung bei der Überwachung eines Gewebes (1825), wobei die Vorrichtung (1800) derart konfiguriert ist, dass es einen Gewebeteil während der Überwachung hält und charakterisiert, wobei diese Vorrichtung (1800) ein Gehäuse (1810) und eine Gewebetyp-Sensoreinheit (1830) umfasst, die auf dem Gehäuse (1810) montiert ist, wobei die Vorrichtung **dadurch gekennzeichnet ist, dass** das Gehäuse (1810) eine innere Oberfläche und eine äußere Oberfläche aufweist, und dass die Gewebetyp-Sensoreinheit (1830) entweder auf einer oder auf beiden dieser inneren und äußeren Oberflächen montiert ist, wobei das Gehäuse (1810) eine der folgenden Konfigurationen zum Halten des zu überwachenden Gewebeteils aufweist:
(1) Das Gehäuse (1810) ist derart konfiguriert, dass es den Gewebeteil umschließt, der in das Gehäuse eingeführt wird, und es trägt die Gewebetyp-Sensoreinheit (1830), die mindestens auf dessen innerer Oberfläche montiert ist; oder
(2) das Gehäuse (1810) trägt die Gewebetyp-Sensoreinheit (1830), die mindestens auf der äußeren Oberfläche des Gehäuses (1810) montiert ist, und die zu einem Teil mit einem Befestigungsmechanismus zum Befestigen des Gewebeteils an der äußeren Oberfläche des Gehäuses verbunden ist, wodurch bewirkt wird, dass der Gewebeteil, der die äußere Oberfläche des Gehäuses (1810) umgibt, diesen Teil des Gehäuses umhüllt;
wobei die Vorrichtung (1800) somit konfiguriert und betriebsbereit zur Charakterisierung des Gewebeteils gemacht wird, der in dem Gehäuse (1810) gehalten wird.

2. Vorrichtung (1800) nach Anspruch 1, wobei die Sensoreinheit eine Vielzahl von Sensorelementen umfasst.

3. Vorrichtung (1800) nach Anspruch 2, wobei die Sensorelemente innerhalb der Oberfläche des Gehäuses (1810) verteilt sind.

4. Vorrichtung (1800) nach Anspruch 1 oder 2, umfassend einen Roboterarm zum Rotieren dieser Sensoreinheit in Bezug auf das Gehäuse (1810).

5. Vorrichtung (1800) nach Anspruch 1 oder 2, umfassend einen Roboterarm zum Rotieren des Gehäuses (1810)in Bezug auf diese Sensoreinheit.

6. Vorrichtung (1800) nach einem der vorherigen Ansprüche, wobei das Gehäuse (1810) einen Körper aufweist, der eine der folgenden Konfigurationen aufweist: ein starrer Körper, ein flexibler Körper, ein dehnbarer Körper und ein erweiterbarer Körper.

7. Vorrichtung (1800) nach Anspruch 6, wobei der Körper des Gehäuses (1810) die Form einer anatomischen Besonderheit aufweist.

8. Vorrichtung (1800) nach einem der vorherigen Ansprüche, umfassend mindestens ein Bündel zur Signalübermittlung zur Übertragung und zum Empfang von Signalen an den und vom Sensor.

9. Vorrichtung (1800) nach einem der vorherigen Ansprüche, wobei das Gehäuse (1810) derart konfiguriert ist, dass es den Gewebeteil umschließt, wobei die Vorrichtung einen Befestigungsmechanismus umfasst, der derart konfiguriert ist, dass er den Gewebeteil an der inneren Oberfläche des Gehäuses (1810) befestigt.

10. Vorrichtung (1800) nach einem der vorherigen Ansprüche, wobei der Befestigungsmechanismus ein Vakuumsystem umfasst.

11. Vorrichtung (1800) nach Anspruch 10, das eine Anschlussstelle zu einer Vakuumquelle umfasst.

12. Vorrichtung (1800) nach Anspruch 11, wobei das Gehäuse (1810) eine oder mehrere Öffnungen umfasst, um eine Vakuumkommunikation zwischen diesem Gewebe und dieser Vakuumquelle herzustellen.

13. Vorrichtung (1800) nach einem der vorherigen Ansprüche, das derart konfiguriert ist, dass es das Gewebe (1825) überwacht, während es im Körper eines Probanden ist, oder derart, dass es das entfernte Gewebe (1825) außerhalb des Körpers des Probanden überwacht.

14. Vorrichtung (1800) nach Anspruch 13, das derart konfiguriert ist, dass es das Gewebe (1825) während der Entfernung des Gewebeteils aus dem Körper eines Probanden überwacht.

15. Vorrichtung (1800) nach einem der vorherigen Ansprüche, wobei das Gehäuse (1810) derart konfiguriert ist, dass es von dem Gewebeteil umgeben wird oder diesen Gewebeteil umschließt, da es die Form einer Kavität oder eines geschlossenen Lumens aufweist.

16. System zur Verwendung bei der Überwachung eines Gewebes (1825), wobei das System folgendes umfasst:
- eine Vorrichtung (1800) nach einem der vorherigen Ansprüche; und
- ein computerbasiertes System (95), das mit dieser Vorrichtung (1800) verbunden werden kann, wobei das computerbasierte System (95) folgendes umfasst:
∘ Ein Analyseprogramm, das derart konfiguriert und betriebsbereit ist, dass es Daten analysiert, die durch die Gewebetyp-Sensoreinheit (1830) erzeugt werden, wobei es feststellt, ob ein sauberer Rand (24) gesunden Gewebes in einer peripheren Region des Gewebeteils vorhanden ist, und das Daten erzeugt, welche die Analyseergebnisse anzeigen; und
∘ Eine Ausgabevorrichtung, die Ausgabedaten zur Verfügung stellt, die den Daten, die durch das Analyseprogramm erzeugt werden, entsprechen.

## Revendications

1. Dispositif (1800) destiné à être utilisé pour contrôler un tissu (1825), ce dispositif (1800) étant conçu pour maintenir et caractériser un morceau de tissu pendant le contrôle, ledit dispositif (1800) comprenant un boîtier (1810) et une unité de détection de type de tissu (1830) montée sur le boîtier (1810), le dispositif étant **caractérisé en ce que** ledit boîtier (1810) a une surface intérieure et une surface extérieure et que l'unité de détection de type de tissu (1830) est montée sur l'un ou l'autre ou les deux desdites surfaces intérieure et extérieure, le boîtier (1810) ayant une des configurations suivantes pour maintenir le morceau de tissu qui est contrôlé :
(1) le boîtier (1810) est conçu pour renfermer le morceau de tissu inséré dans le boîtier et supporte l'unité de détection de type de tissu (1830) montée au moins sur sa surface intérieure ; ou
(2) le boîtier (1810) supporte l'unité de détection de type de tissu (1830) montée au moins sur la surface extérieure du boîtier (1810) dont une partie est associée à un mécanisme de fixation destiné à fixer le morceau de tissu à la surface extérieure du boîtier en amenant le morceau de tissu entourant la surface extérieure du boîtier (1810) à envelopper ladite partie du boîtier ;
le dispositif (1800) étant ainsi conçu et actionnable pour caractériser le morceau de tissu maintenu par le boîtier (1810).

2. Dispositif (1800) selon la revendication 1, dans lequel l'unité de détection comprend une pluralité d'éléments de détection.

3. Dispositif (1800) selon la revendication 2, dans lequel lesdits éléments de détection sont répartis dans la surface du boîtier (1810).

4. Dispositif (1800) selon la revendication 1 ou 2, comprenant un bras robotisé pour faire tourner ladite unité de détection par rapport au boîtier (1810).

5. Dispositif (1800) selon la revendication 1 ou 2, comprenant un bras robotisé pour faire tourner ladite unité de détection par rapport au boîtier (1810) à ladite unité de détection.

6. Dispositif (1800) selon l'une quelconque des revendications précédentes, dans lequel le boîtier (1810) possède un corps ayant une des configurations suivantes : un corps rigide, un corps souple, un corps étirable et un corps expansible.

7. Dispositif (1800) selon la revendication 6, dans lequel le corps du boîtier (1810) a la forme d'une structure anatomique.

8. Dispositif (1800) selon l'une quelconque des revendications précédentes, comprenant au moins un faisceau de communication de signaux pour transmettre et recevoir des signaux depuis et vers l'unité de détection.

9. Dispositif (1800) selon l'une quelconque des revendications précédentes, dans lequel dans lequel le boîtier (1810) est conçu pour renfermer le morceau de tissu, le dispositif comprenant un mécanisme de fixation conçu pour fixer le morceau de tissu à la surface intérieure du boîtier (1810).

10. Dispositif (1800) selon l'une quelconque des revendications précédentes, dans lequel le mécanisme de fixation comprend un système de dépressurisation.

11. Dispositif (1800) selon la revendication 10, comprenant un connecteur à une source de vide.

12. Dispositif (1800) selon la revendication 11, dans lequel ledit boîtier (1810) comprend une ou plusieurs ouvertures pour permettre la communication de vide entre ledit tissu et ladite source de vide.

13. Dispositif (1800) selon l'une quelconque des revendications précédentes, conçu pour contrôler le tissu (1825) pendant qu'il se trouve dans le corps d'un sujet ou pour contrôler le tissu extrait (1825) en dehors du corps du sujet.

14. Dispositif (1800) selon la revendication 13, conçu pour contrôler le tissu (1825) pendant l'extraction du morceau de tissu du corps d'un sujet.

15. Dispositif (1800) selon l'une quelconque des revendications précédentes, dans lequel le boîtier (1810) est conçu pour être entouré par ou pour renfermer le morceau de tissu ayant la forme d'une cavité ou d'un lumen fermé.

16. Système destiné à être utilisé pour contrôler un tissu (1825), ce système comprenant
- un dispositif (1800) selon l'une quelconque des revendications précédentes ; et
- un système informatisé (95) connectable audit dispositif (1800), ce système informatisé (95) comprenant :
∘ un équipement d'analyse conçu et actionnable pour analyser des données générées par l'unité de détection de type de tissu (1830) en déterminant si une marge nette (24) de tissu sain existe dans une zone périphérique du morceau de tissu et en générant des données indiquant les résultats d'analyse : et
∘ un dispositif d'édition qui fournit des données d'édition correspondant auxdites données générées par l'équipement d'analyse.
